# EUROPEAN PATENT APPLICATION

(11) **EP 4 398 261 A1**
(43) Date of publication of application: **10.07.2024**
(21) Application number: 22864390.4
(22) Date of filing: 25.08.2022
(51) Int. Cl.: G16H 50/30, G06F 3/01, G06F 3/04815

(54) **STEREOGNOSTIC CAPABILITY EVALUATION SYSTEM, STEREOGNOSTIC CAPABILITY EVALUATION DEVICE, STEREOGNOSTIC CAPABILITY EVALUATION PROGRAM, AND STEREOGNOSTIC CAPABILITY EVALUATION METHOD**

(30) Priority: 31.08.2021 JP 2021141500; 01.03.2022 JP 2022030855
(71) Applicant: Sumitomo Pharma Co., Ltd., Osaka 541-0045 (JP)
(72) Inventor: OCHIAI, Yasushi, Tokyo 103-6012 (JP); KASAI, Kazuki, Tokyo 158-0094 (JP); EGAMI, Shin, Takatsuki-shi, Osaka 569-1123 (JP)
(74) Representative: Willett, Christopher David
(86) International application number: PCT/JP2022/032035
(87) International publication number: WO 2023/032806

(57) **Abstract**

Objective evaluation of stereognostic ability is achieved. A stereognostic ability evaluation system (100) evaluates a stereognostic ability of a subject based on a positional change of a moving object (Cs1) moving as operated by the subject. A position acquisition unit (101b) acquires information about the position of the moving object (Cs1). A stereognostic ability determination unit (101e) determines the stereognostic ability of the subject from at least one of a first feature value and a second feature value, the first feature value being derived from a value obtained by differentiating a first distance between a reference object and the moving object (Cs1) with time at least once, the reference object preceding the moving object (Cs1) in a direction in which the moving object (Cs1) travels, the second feature value being derived from a second distance between a reference line and the moving object (Cs1), the reference line extending in the direction in which the moving object (Cs1) travels.

## Description

### TECHNICAL FIELD

The present disclosure relates to a stereognostic ability evaluation system, a stereognostic ability evaluation device, a stereognostic ability evaluation program, and a stereognostic ability evaluation method.

### BACKGROUND ART

A conventionally known system evaluates a cognitive function of a subject based on a reaction of the subject. For example, Japanese Patent No. 6000968 (PTL 1) discloses a system using a portable touch-screen personal computing device as a system for evaluating a cognitive function using a visual measurement. The system performs a cognitive assessment for an individual based on a speed of a response to a displayed stimulus for cognitive assessment. In a cognitive assessment test, a response time from when a character is displayed on a display of the personal computing device to a user's response, or when a button is pressed, is measured.

Japanese National Patent Publication No. 2015-502238 (PTL 2) discloses a system for mapping a peripheral vision of a subject for providing a video game, including a test in which the subject finds a visual stimulus presented for a short period of time. In this system, the display displays a target, and a glaucomatous visual field defect is measured based on a user's response to the target.

### CITATION LIST

### PATENT LITERATURE

PTL 1: Japanese Patent No. 6000968
PTL 2: Japanese National Patent Publication No. 2015-502238

### SUMMARY OF INVENTION

### TECHNICAL PROBLEM

Stereognostic ability is an ability to recognize whether an object is near or far, and to respond appropriately thereto, and may also be represented for example as perspective. In recent years, it has been known that when elderly people or the like have an impaired cognitive function, they also tend to have a similarly impaired stereognostic ability. Accordingly, evaluating a stereognostic ability may have an effect similar to that of evaluating a cognitive function.

However, in the system disclosed in PTL 1, while a displayed object is moved, the user's perspective with respect to the object is not considered. Further, in this system, a result of a measurement of a cognitive function also depends on a variety of types of attributes such as the subject's level of concentration, the subject's familiarity with operating the system, etc., and it is thus difficult to objectively evaluate the user's stereognostic ability. Further, while the system disclosed in PTL 2 measures a distance between the subject and a monitor, the system does not consider the subject's perspective with respect to the displayed target.

The present disclosure has been made to solve the above problems, and an object of the present disclosure is to implement objective evaluation of stereognostic ability.

### SOLUTION TO PROBLEM

According to an aspect of the present disclosure, a stereognostic ability evaluation system evaluates a stereognostic ability of a subject based on a positional change of a moving object moving as operated by the subject. The stereognostic ability evaluation system comprises a position acquisition unit and a stereognostic ability determination unit. The position acquisition unit acquires information about the position of the moving object. The stereognostic ability determination unit determines the stereognostic ability of the subject from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

According to another aspect of the present disclosure, a stereognostic ability evaluation system evaluates a stereognostic ability of a subject based on a behavioral change of a moving object moving as operated by the subject. The stereognostic ability evaluation system comprises a behavior acquisition unit and a stereognostic ability determination unit. The behavior acquisition unit acquires information about the behavior of the moving object. The stereognostic ability determination unit determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.

According to another aspect of the present disclosure a stereognostic ability evaluation device evaluates a stereognostic ability of a subject based on a positional change of a moving object moving as operated by the subject. The stereognostic ability evaluation device comprises a position acquisition unit, a stereognostic ability determination unit, and a housing. The position acquisition unit acquires information about the position of the moving object. The stereognostic ability determination unit determines the stereognostic ability of the subject from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels. The housing accommodates the position acquisition unit and the stereognostic ability determination unit.

According to another aspect of the present disclosure a stereognostic ability evaluation device evaluates a stereognostic ability of a subject based on a behavioral change of a moving object moving as operated by the subject. The stereognostic ability evaluation device comprises a behavior acquisition unit, a stereognostic ability determination unit, and a housing. The behavior acquisition unit acquires information about the behavior of the moving object. The stereognostic ability determination unit determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels. The housing accommodates the behavior acquisition unit and the stereognostic ability determination unit.

According to another aspect of the present disclosure a stereognostic ability evaluation program is executed by a computer to cause the computer to configure a stereognostic ability evaluation device to evaluate a stereognostic ability of a subject based on a positional change of a moving object moving as operated by the subject. The stereognostic ability evaluation device comprises a position acquisition unit and a stereognostic ability determination unit. The position acquisition unit acquires information about the position of the moving object. The stereognostic ability determination unit evaluates the stereognostic ability of the subject from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

According to another aspect of the present disclosure a stereognostic ability evaluation program is executed by a computer to cause the computer to configure a stereognostic ability evaluation device to evaluate a stereognostic ability of a subject based on a behavioral change of a moving object moving as operated by the subject. The stereognostic ability evaluation device comprises a behavior acquisition unit and a stereognostic ability determination unit. The behavior acquisition unit acquires information about the behavior of the moving object. The stereognostic ability determination unit evaluates the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.

According to another aspect of the present disclosure a stereognostic ability evaluation method evaluates a stereognostic ability of a subject based on a positional change of a moving object moving as operated by the subject. The method comprises obtaining information about a position of the moving object. The method further comprises determining the stereognostic ability of the subject from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

According to another aspect of the present disclosure a stereognostic ability evaluation method evaluates a stereognostic ability of a subject based on a behavioral change of a moving object moving as operated by the subject. The method comprises obtaining information about a behavior of a moving object. The method further comprises determining the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.

### ADVANTAGEOUS EFFECTS OF INVENTION

According to the presently disclosed stereognostic ability evaluation system, stereognostic ability evaluation device, stereognostic ability evaluation program, and stereognostic ability evaluation method, an objective evaluation of a stereognostic ability of a subject can be implemented by determining the stereognostic ability of the subject from at least one of a first feature value and a second feature value, the first feature value being derived from a value obtained by differentiating a first distance between a reference object and a moving object with time at least once, the second feature value being relevant to a second distance between a reference line and the moving object.

### BRIEF DESCRIPTION OF DRAWINGS

Fig. 1 is a block diagram showing a configuration of a stereognostic ability evaluation system according to a first embodiment.
Fig. 2 is a diagram of a hardware configuration of the stereognostic ability evaluation system of Fig. 1.
Fig. 3 is a functional block diagram of the processor of Fig. 2 showing a configuration of a function to evaluate a stereognostic ability.
Fig. 4 is a functional block diagram of the processor of Fig. 2 showing a configuration of a machine learning function.
Fig. 5 is a diagram for illustrating a distance relating to an automobile necessary for deriving a feature value input to the evaluation model of Fig. 2.
Fig. 6 is a timing plot in velocity of a preceding automobile and the automobile of Fig. 5.
Fig. 7 is a timing plot representing a result of determining in the timing plot of Fig. 6 a segment in which the preceding automobile is traveling with constant velocity.
Fig. 8 is a table indicating a plurality of feature values derived from traveling data of a plurality of segments.
Fig. 9 is a diagram representing together a trajectory of the preceding automobile and that of the automobile in Fig. 5 when the automobile is driven by a driver provided with a label indicating a high stereognostic ability.
Fig. 10 is a diagram representing together a trajectory of the preceding automobile and that of the automobile in Fig. 5 when the automobile is driven by a driver provided with a label indicating a low stereognostic ability.
Fig. 11 is a diagram of an example of the decision tree in Fig. 2, showing a decision tree including a feature value derived from an interval acceleration as an explanatory variable.
Fig. 12 is a diagram showing a classification result when a driver having a low stereognostic ability is added to a classification target of the decision tree of Fig. 11.
Fig. 13 is a diagram of another example of the decision tree in Fig. 2, showing a decision tree DT2 including feature values derived from a slide amplitude in absolute value and a slide velocity in absolute value as explanatory variables.
Fig. 14 is a diagram of another example of the decision tree in Fig. 2, showing a decision tree including feature values derived from a slide amplitude in absolute value, a slide velocity in absolute value, and a slide acceleration in absolute value as explanatory variables.
Fig. 15 is a diagram of another example of the decision tree in Fig. 2, showing a decision tree including feature values derived from a slide amplitude in absolute value, a slide velocity in absolute value, and a slide acceleration in absolute value as explanatory variables.
Fig. 16 is a diagram of another example of the decision tree in Fig. 2, showing a decision tree including a feature value derived from a slide amplitude as an explanatory variable.
Fig. 17 is a diagram of another example of the decision tree in Fig. 2, showing a decision tree including a feature value derived from a slide amplitude as an explanatory variable.
Fig. 18 is a flowchart of a process in a stereognostic ability evaluation method performed by a processor that executes the stereognostic ability evaluation program of Fig. 2.
Fig. 19 is a block diagram showing a configuration of a stereognostic ability evaluation system according to an exemplary variation of the first embodiment.
Fig. 20 is a diagram showing a hardware configuration of the stereognostic ability evaluation system of Fig. 19.
Fig. 21 is a diagram showing a state in which a subject has his/her stereognostic ability measured in a measurement test through a drive simulation in a stereognostic ability evaluation system according to a second embodiment.
Fig. 22 is a diagram showing an example of a screen displayed for a subject via a stereognostic ability evaluation device in the drive simulation by the stereognostic ability evaluation system of Fig. 21.
Fig. 23 is an external perspective view of the stereognostic ability evaluation device of Fig. 21.
Fig. 24 is a diagram showing a hardware configuration of the stereognostic ability evaluation device of Fig. 23.
Fig. 25 is a diagram representing a concept of a visual target used for measuring a function of an eye.
Fig. 26 is a diagram representing a relationship between a visual target's distance (a distance between an eye of a subject and the visual target) and pupillary diameter through near pupillary reflex.
Fig. 27 is a diagram representing a relationship between a visual target's distance and pupillary position (or vergence).
Fig. 28 is a functional block diagram of the processor of Fig. 24 showing a configuration of a function to evaluate a stereognostic ability.
Fig. 29 is a flowchart of a process in a stereognostic ability evaluation method performed by a processor that executes the stereognostic ability evaluation program in Fig. 24.
Fig. 30 is a diagram showing a hardware configuration of a stereognostic ability evaluation system according to a third embodiment.
Fig. 31 is a functional block diagram of the processor of Fig. 30 showing a configuration of a function to evaluate a stereognostic ability.
Fig. 32 is a functional block diagram of the processor of Fig. 30 showing a configuration of a machine learning function.
Fig. 33 is a diagram showing a hardware configuration of a stereognostic ability evaluation system according to an exemplary variation of the third embodiment.

### DESCRIPTION OF EMBODIMENTS

Hereinafter, embodiments of the present disclosure will be described in detail with reference to the accompanying drawings. In the figures, identical or corresponding components are identically denoted and in principle will not be described repeatedly.

### [First Embodiment]

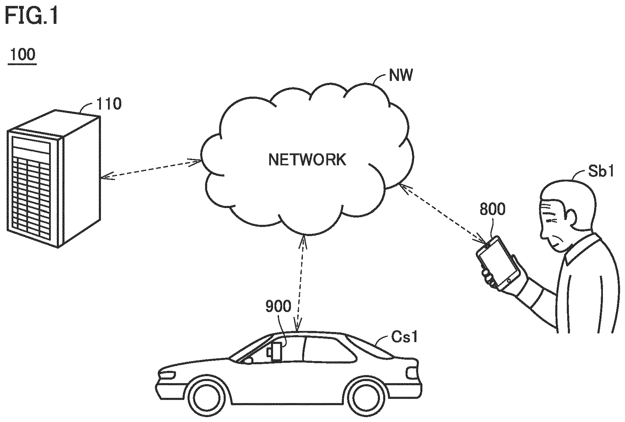

Fig. 1 is a block diagram showing a configuration of a stereognostic ability evaluation system 100 according to a first embodiment. As shown in Fig. 1, stereognostic ability evaluation system 100 comprises an information processing device 110 (a stereognostic ability evaluation device), a terminal device 800 of a subject Sb1, and a drive recorder 900 (a movement recording device) mounted in an automobile Cs1. Information processing device 110, terminal device 800, automobile Cs1, and drive recorder 900 are interconnected via a network NW. Network NW includes, for example, the Internet, a LAN (Local Area Network), or a cloud system.

Subject Sb1 is a subject whose stereognostic ability is measured by stereognostic ability evaluation system 100. Subject Sb1 gets in automobile Cs1 and drives automobile Cs1. Traveling data (or video data) of automobile Cs1 driven by subject Sb1 is recorded by drive recorder 900.

Information processing device 110 acquires the traveling data of subject Sb1 from drive recorder 900. Information processing device 110 derives from the traveling data an indicator (a feature value) that characterizes a positional change of automobile Cs1 as it moves as operated by subject Sb1. Information processing device 110 evaluates subject Sb1's stereognostic ability based on the feature value. Information processing device 110 transmits the evaluation result of the stereognostic ability to terminal device 800 of subject Sb1. Information processing device 110 includes, for example, a personal computer or a workstation. Information processing device 110 may be mounted in automobile Cs1.

Subject Sb1 can confirm the evaluation result by referring to terminal device 800 wherever the subject may be insofar as connection between terminal device 800 and network NW can be established. Terminal device 800 includes, for example, a smartphone capable of wireless communication.

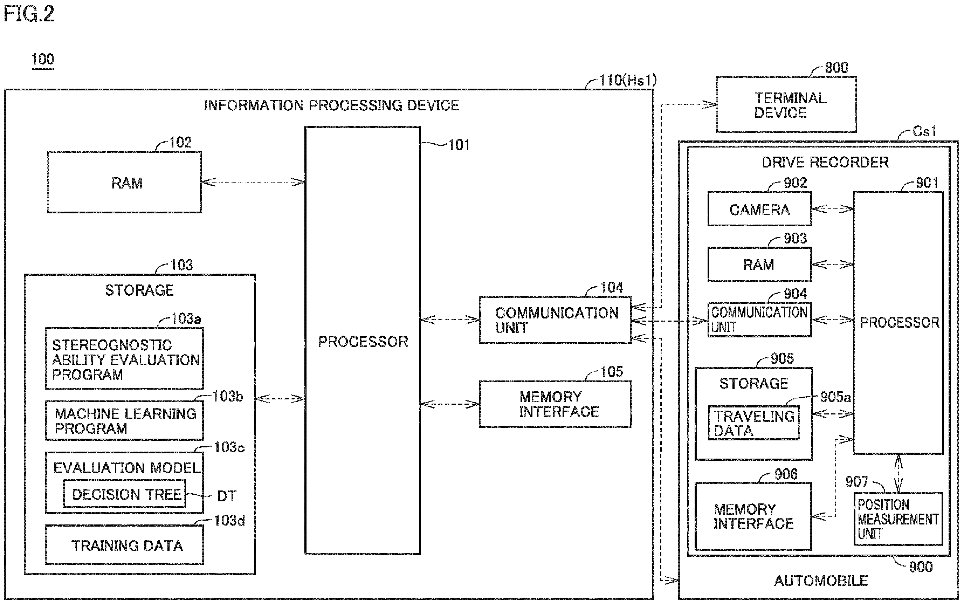

Fig. 2 is a diagram showing a hardware configuration of stereognostic ability evaluation system 100 of Fig. 1. As shown in Fig. 2, information processing device 110 includes a processor 101, a random access memory (RAM) 102, a storage 103, a communication unit 104, a memory interface 105, and a housing Hs1. Housing Hs1 accommodates processor 101, random access memory (RAM) 102, storage 103, communication unit 104, and memory interface 105.

Processor 101 is processing circuitry for executing a variety of types of functions for controlling an operation of information processing device 110. Processor 101 includes a CPU (Central Processing Unit) or a GPU (Graphics Processing Unit) that operates an information device such as a computer.

RAM 102 includes a volatile memory. RAM 102 is used as a work area when processor 101 operates, a temporary data storage area, or the like.

Storage 103 includes a non-volatile memory such as a flash ROM (Read Only Memory) or an SSD (Solid State Drive). Storage 103 stores a computer program, and data referred to when the computer program is executed. For example, an OS (Operating System) program (not shown), a stereognostic ability evaluation program 103a, and a machine learning program 103b are stored as the computer program in storage 103. Storage 103 also stores an evaluation model 103c (a specific model) and training data 103d as data referred to when the computer program is executed.

Evaluation model 103c evaluates subject Sb1's stereognostic ability from a feature value derived from the traveling data of subject Sb1. Evaluation model 103c may be a classification model that classifies subject Sb1's stereognostic ability into any one of a plurality of predetermined levels, or may be a regression model that outputs a numerical value corresponding to the stereognostic ability. Evaluation model 103c is referred to by stereognostic ability evaluation program 103a. Evaluation model 103c includes a decision tree DT (Decision Tree). Evaluation model 103c may include, for example, a neural network or a support vector machine. Training data 103d is referred to by machine learning program 103b. Machine learning program 103b executes, for example, CART (Classification and Regression Trees) as a machine learning algorithm. Training data 103d includes traveling data (or teacher data) of a driver previously labeled by the driver's cognitive ability. Labels attached to the traveling data may for example be a level of stereognostic ability determined in advance from a mental rotation task, a visual ability evaluation test, a "visual ability" comprehension test, the K-ABC (Kaufman Assessment Battery for Children) test, Test of Visual Perceptual Skills, a motion-excluding visual cognition test and other similar existing assessment batteries for visual perception of space, a driver's driving skill, occupation, age, or driving history (information about an attribute of a subject), etc.

Communication unit 104 communicates with terminal device 800, automobile Cs1, and drive recorder 900 via network NW. Communication unit 104 and network NW may be connected by wired communication (for example, Ethernet^{®}) or wireless communication (for example, Wi-Fi^{®}).

Memory interface 105 allows access to a storage medium external to information processing device 110. Memory interface 105 corresponds to an SD (Secure Digital) card and a USB (Universal Serial Bus).

An input/output unit (not shown) that receives an input from a user and displays a processing result of processor 101 to the user may be connected to information processing device 110. The input/output unit includes, for example, a display, a touch panel, a keyboard, a speaker, a lamp, and a mouse.

Drive recorder 900 includes a processor 901, a camera 902, a RAM 903, a communication unit 904, a storage 905, a memory interface 906, and a position measurement unit 907. Processor 901, RAM 903, communication unit 904 and memory interface 906 are substantially identical in function to processor 101, RAM 102, communication unit 104 and memory interface 105 of information processing device 110, and accordingly, will not be described in function repeatedly.

Camera 902 generates traveling data 905a as viewed at the driver's seat of automobile Cs1 in the direction in which automobile Cs1 travels. Traveling data 905a is stored in storage 905. Traveling data 905a is transmitted to information processing device 110 by communication unit 904. Position measurement unit 907 measures the position of automobile Cs1 using GNSS (Global Navigation Satellite System) such as GPS (Global Positioning System).

Automobile Cs1 transmits data about a reaction of subject Sb1 operating automobile Cs1 to information processing device 110. Specifically, automobile Cs1 transmits data about the reaction of subject Sb1 to information processing device 110 based on information input by the subject to the handle, the accelerator pedal, the brake pedal, etc. (for example, a rotation angle of the handle, an angle by which the accelerator pedal is pressed, and an angle by which the brake pedal is pressed). The term "reaction" means recognizing that a moving object is near/far and responding accordingly.

Stereognostic ability evaluation system 100 has a stereognostic ability evaluation function implemented by processor 101 reading stereognostic ability evaluation program 103a stored in storage 103, and executing the program using a work area of RAM 102. When stereognostic ability evaluation program 103a is executed, a module implementing a variety of types of functions for stereognostic ability evaluation is formed and performs an operation implementing the functions.

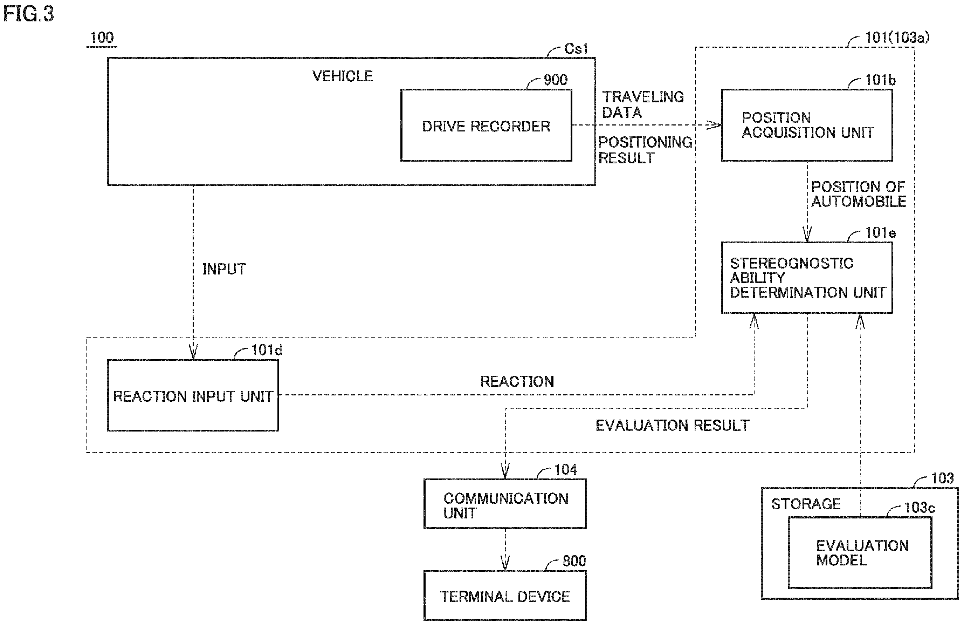

Fig. 3 is a functional block diagram of processor 101 of Fig. 2 showing a configuration of a function to evaluate a stereognostic ability. In stereognostic ability evaluation system 100, processor 101 executes stereognostic ability evaluation program 103a stored in storage 103 to configure a module forming functional blocks referred to as a position acquisition unit 101b, a reaction input unit 101d, and a stereognostic ability determination unit 101e. Accordingly, in place of processor 101 and stereognostic ability evaluation program 103a shown in Fig. 2, Fig. 3 shows functional blocks implemented thereby. These functional blocks will be described below.

Position acquisition unit 101b obtains the traveling data of subject Sb1 and a result of a measurement of a position of automobile Cs1 (or a positioning result) from drive recorder 900. Position acquisition unit 101b acquires information about the position of automobile Cs1 from the traveling data and the positioning result. Position acquisition unit 101b outputs information about the position of automobile Cs1 to stereognostic ability determination unit 101e. The information about the position of automobile Cs1 includes information capable of deriving at least one of a value (an interval velocity) obtained by differentiating an inter-vehicle distance between automobile Cs1 and another preceding automobile (a preceding automobile) with time once, a value (an interval acceleration) obtained by differentiating the distance with time twice, a distance (a slide amplitude) between a reference line extending in the direction in which automobile Cs1 travels and automobile Cs1, a value (a lateral velocity) obtained by differentiating the slide amplitude with time once, and a value (a slide acceleration) obtained by differentiating the slide amplitude with time twice. For example, the information about the position of automobile Cs1 includes information that can determine a correspondence between time and automobile Cs1's three-dimensional spatial coordinates, the three-dimensional spatial coordinates of each of at least one reference position, and a reference line in a three-dimensional space.

Reaction input unit 101d receives an input of an active reaction made by subject Sb1 to correspond to a three-dimensional position of automobile Cs1 as recognized by subject Sb1. Reaction input unit 101d determines the reaction of subject Sb1 driving automobile Cs1, based on information input by the subject to the handle, accelerator pedal, brake pedal, etc. of automobile Cs1. Reaction input unit 101d outputs information about the reaction to stereognostic ability determination unit 101e.

The active reaction means an active operation for achieving a predetermined goal, and specifically means an operation performed for a target whose position is dynamically determined by the position of automobile Cs1. The active reaction includes, for example, an operation causing automobile Cs1 to approach a predetermined site. In this case, a difference between the position of automobile Cs1 and the location of the predetermined site is dynamically determined by the position of automobile Cs1, and decreasing the difference will be a goal. Further, the active reaction includes, for example, an operation to constantly maintain a distance between automobile Cs1 and another automobile traveling in advance (a preceding automobile). In this case, a difference between the preceding automobile and automobile Cs1 is dynamically determined by the position of the preceding automobile, and constantly keeping the difference will be a goal.

Based on the information about the position of automobile Cs1, stereognostic ability determination unit 101e derives a feature value derived from at least one of an interval velocity of automobile Cs1, an interval acceleration thereof, a slide amplitude in absolute value thereof, a slide velocity in absolute value thereof, a slide acceleration in absolute value thereof, the slide amplitude, the slide velocity, and the slide acceleration. Stereognostic ability determination unit 101e uses evaluation model 103c to determine subject Sb1's stereognostic ability from the feature value. The information about the reaction received from reaction input unit 101d may be used to determine subject Sb1's stereognostic ability. Stereognostic ability determination unit 101e transmits an evaluation result of the stereognostic ability to terminal device 800 of subject Sb1 via communication unit 104.

In stereognostic ability evaluation system 100, when machine learning program 103b is executed by processor 101, information processing device 110 functions as a training device to train evaluation model 103c to be a trained evaluation model.

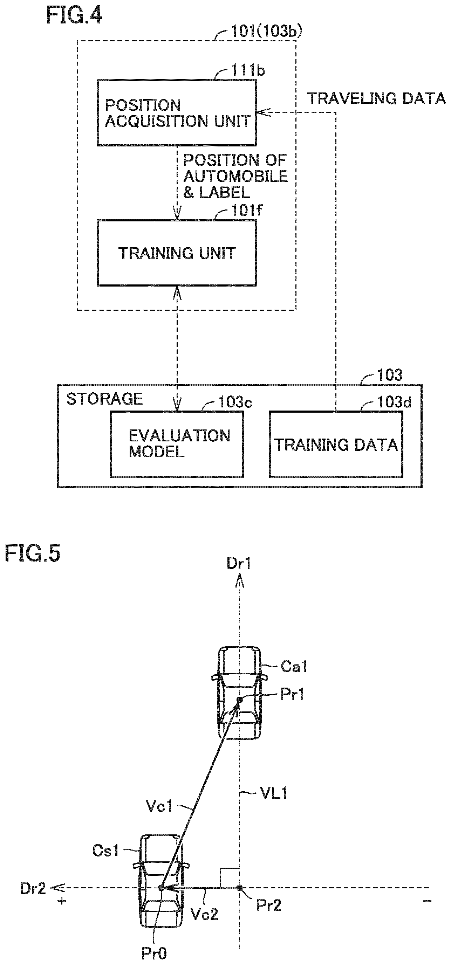

Fig. 4 is a functional block diagram of processor 101 of Fig. 2 showing a configuration of a machine learning function. As shown in Fig. 4, in stereognostic ability evaluation system 100, machine learning program 103b is executed by processor 101 to configure a module forming functional blocks referred to as a position acquisition unit 111b and a training unit 101f. Therefore, in place of processor 101 and machine learning program 103b shown in Fig. 2, Fig. 4 shows functional blocks implemented thereby. These functional blocks will now be described below.

Position acquisition unit 111b acquires traveling data from training data 103d. As well as position acquisition unit 101b of Fig. 3, position acquisition unit 111b acquires information about a position of an automobile corresponding to the traveling data. Position acquisition unit 111b outputs information about the position of the automobile and a label attached to the traveling data to training unit 101f.

Based on the information about the position of the automobile acquired from position acquisition unit 111b, training unit 101f derives a feature value derived from at least one of the automobile's interval velocity, interval acceleration, slide amplitude in absolute value, slide velocity in absolute value, slide acceleration in absolute value, slide amplitude, slide velocity, and slide acceleration. Training unit 101f uses the feature value and the label attached to the information about the position of the automobile to subject evaluation model 103c to machine learning to train evaluation model 103c. For example, training unit 101f executes the CART to generate a decision tree DT such that a driver's stereognostic ability corresponding to the feature value serves as an objective variable and the feature value serves as an explanatory variable.

When an interval between a reference object and a moving object is defined as a first distance and a distance (a slide amplitude) between a reference line extending in the direction in which the moving object travels and the moving object is defined as a second distance, the present disclosure includes a manner of implementation in the following scope.

That is, the presently disclosed stereognostic ability evaluation system evaluates a stereognostic ability of a subject based on a positional change of a moving object moving as operated by the subject. The stereognostic ability evaluation system comprises a position acquisition unit and a stereognostic ability determination unit. The position acquisition unit acquires information about the position of the moving obj ect.

The stereognostic ability determination unit determines the stereognostic ability of the subject from at least one of a first feature value and a second feature value, the first feature value being relevant to a first distance between a reference object preceding the moving object in the direction in which the moving object travels and the moving object, the second feature value being relevant to a second distance between a reference line extending in the direction in which the moving object travels and the moving object.

Furthermore, the position acquisition unit acquires information about a position of the moving object, and at least one of the first feature value and the second feature value is derived from the information about the position of the moving object. The first feature value may be derived from any one of a value of the first distance, a value obtained by differentiating the first distance with time once, and a value obtained by differentiating the first distance with time twice. The second feature value may be derived from any one of a value of the second distance, a value obtained by differentiating the second distance with time once, and a value obtained by differentiating the second distance with time twice.

Fig. 5 is a diagram for illustrating a distance for automobile Cs1 necessary for deriving a feature value input to evaluation model 103c of Fig. 2. As shown in Fig. 5, a preceding automobile Ca1 travels before automobile Cs1 driven by subject Sb1. Preceding automobile Ca1 and automobile Cs1 travel in a traveling direction Dr1. A slide direction Dr2 is orthogonal to traveling direction Dr1. Preceding automobile Ca1 includes a reference position Pr1 defined as a position of preceding automobile Ca1. Automobile Cs1 includes a reference position Pr0 defined as a position of automobile Cs1. A distance (a first distance) between automobile Cs1 and preceding automobile Ca1 is defined as a magnitude of a vector Vc1 from reference position Pr0 to reference position Pr1.

A reference line VL1 is parallel to traveling direction Dr1 and passes through reference position Pr1. Reference line VL1 includes a reference position Pr2. A vector Vc2 from reference position Pr2 toward reference position Pr0 is orthogonal to traveling direction Dr1. A distance (a second distance) between automobile Cs1 and reference line VL1 is defined as a value obtained by assigning to a magnitude of vector Vc2 a sign corresponding to a direction of vector Vc2 in slide direction Dr2. That is, when vector Vc2 has a direction in the positive direction of slide direction Dr2, the automobile will have a slide amplitude having a positive value. When vector Vc2 has a direction in the negative direction of slide direction Dr2, the automobile will have a slide amplitude having a negative value. Slide amplitude can be defined even when there is no preceding automobile Ca1. In that case, reference line VL1 is, for example, a center line of a lane on which automobile Cs1 travels. Therefore, even when preceding automobile Ca1 is absent, a feature value derived from a slide amplitude can be used to evaluate a stereognostic ability of subject Sb1.

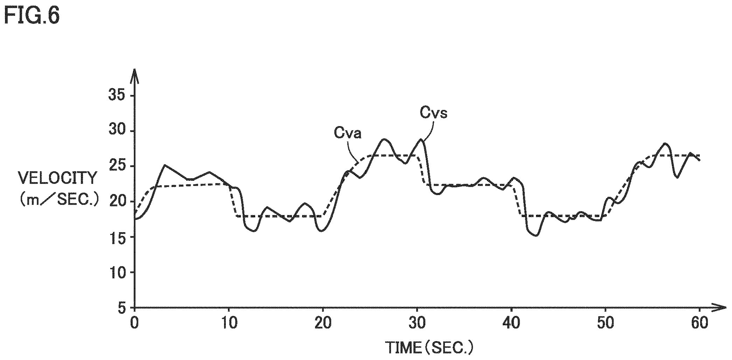

Hereinafter, reference will be made to Figs. 6 to 8 to specifically describe what type of feature value is derived in stereognostic ability evaluation system 100 for evaluating subject Sb1's stereognostic ability. Fig. 6 is a timing plot in velocity of preceding automobile Ca1 and automobile Cs1 in Fig. 5. A graph Cva represents a correspondence between a velocity of preceding automobile Ca1 measured for each sampling time and the sampling time. A graph Cvs represents a correspondence between a velocity of automobile Cs1 measured for each sampling time and the sampling time.

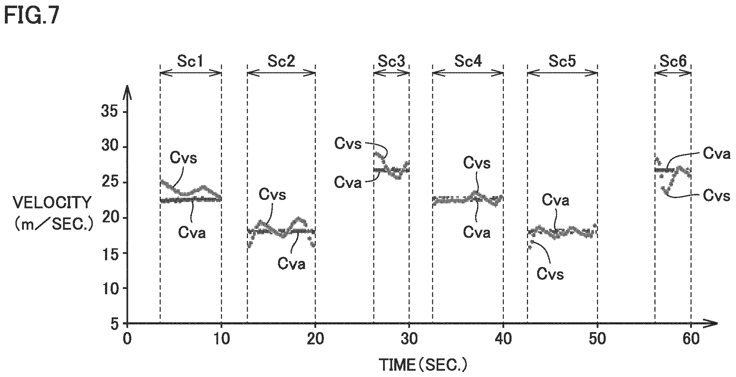

Fig. 7 is a timing plot representing a result of determining a segment in which preceding automobile Ca1 is traveling with constant velocity in the timing plot of Fig. 6. A segment in which preceding automobile Ca1 is traveling with constant velocity is a segment in which a change in velocity of preceding automobile Ca1 is included in a predetermined range. As shown in Fig. 7, a plurality of segments Sc1, Sc2, Sc3, Sc4, Sc5, and Sc6 are identified as segments in which preceding automobile Ca1 is traveling with constant velocity. As a segment in which preceding automobile Ca1 is traveling with constant velocity, 5 or less segments may be extracted, or 7 or more segments may be extracted. Deriving a feature value using traveling data of a segment in which preceding automobile Ca1 is traveling with constant velocity can reduce noise (information less relevant to stereognostic ability) included in the feature value. As a result, subject Sb1's stereognostic ability can be evaluated more accurately. It is also possible to evaluate subject Sb1's stereognostic ability by using a feature value derived from traveling data of a segment in which preceding automobile Ca1 is not driven with constant velocity.

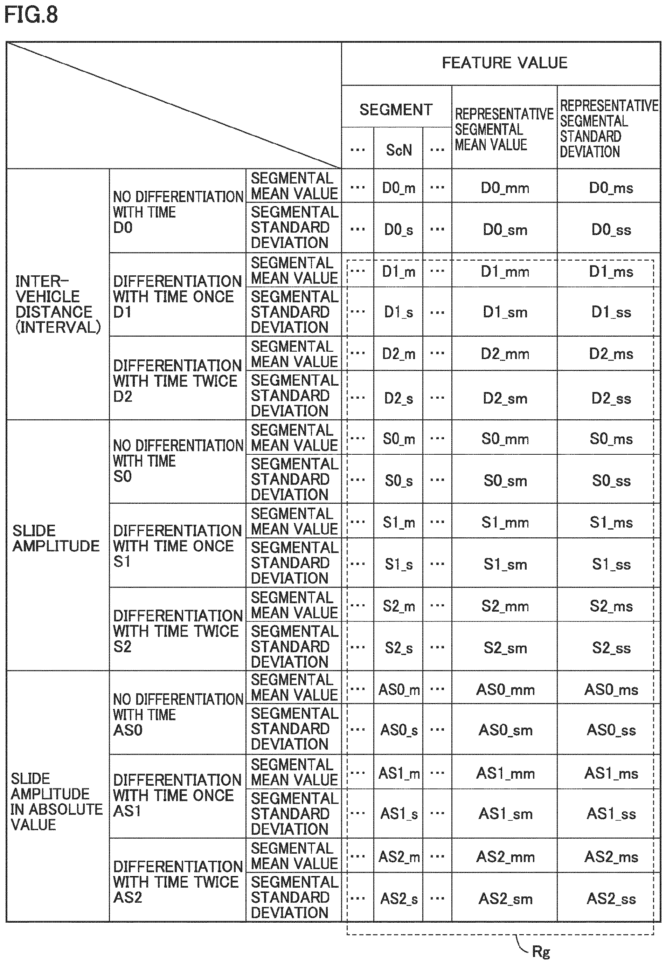

Fig. 8 is a table indicating a plurality of feature values derived from traveling data of a plurality of segments. In the following description, "X_m" represents a mean value (a segmental mean value) of a value X for one segment, and "X_s" represents a standard deviation (a segmental standard deviation) of value X for one segment. "X_mm" represents a mean value (a representative segmental mean value) of segmental mean values (representative segmental values) of value X, and "X_ms" represents a standard deviation (a representative segmental standard deviation) of segmental mean values of value X. "X_sm" represents a mean value (a representative segmental mean value) of segmental standard deviations (representative segmental values) of value X, and "X_ss" represents a standard deviation (a representative segmental standard deviation) of segmental standard deviations of value X. A value X1 represents a value obtained by differentiating value X0 with time once, and a value X2 represents a value obtained by differentiating value X0 with time twice.

As shown in Fig. 8, an inter-vehicle distance D0, an interval velocity D1, an interval acceleration D2, a slide amplitude S0, a slide velocity S1, a slide acceleration S2, slide amplitude S0 in absolute value AS0, the slide velocity in absolute value AS1, and the slide acceleration in absolute value AS2 for each sampling time are calculated from the traveling data for the sampling time.

When a segment ScN is noted, where N is a natural number, a segmental mean value D0_m and a segmental standard deviation D0_s are calculated as feature values for inter-vehicle distance D0. For interval velocity D1, a segmental mean value D1_m and a segmental standard deviation D1_s are calculated as feature values. For interval acceleration D2, a segmental mean value D2_m and a segmental standard deviation D2_s are calculated as feature values.

For slide amplitude S0, a segmental mean value S0_m and a segmental standard deviation S0_s are calculated as feature values. For slide velocity S1, a segmental mean value S1_m and a segmental standard deviation S1_s are calculated as feature values. For slide acceleration S2, a segmental mean value S2_m and a segmental standard deviation S2_s are calculated as feature values.

For slide amplitude S0 in absolute value AS0, a segmental mean value AS0_m and a segmental standard deviation AS0_s are calculated as feature values. For the slide velocity in absolute value AS1, a segmental mean value AS1_m and a segmental standard deviation AS1_s are calculated as feature values. For the slide acceleration in absolute value AS2, a segmental mean value AS2_m and a segmental standard deviation AS2_s are calculated as feature values.

From each segment's segmental mean values D0_m, D1_m and D2_m and segmental standard deviations D0_s, D1_s and D2_s, representative segmental mean values D0_mm, D1_mm, D2_mm, D0_sm, D1_sm, and D2_sm are calculated as feature values, respectively, and representative segmental standard deviations D0_ms, D1_ms, D2_ms, D0_ss, D1_ss, and D2_ss are calculated as feature values, respectively.

From each segment's segmental mean values S0_m, S1_m and S2_m and segmental standard deviations S0_s, S1_s and S2_s, representative segmental mean values S0_mm, S1_mm, S2_mm, S0_sm, S1_sm, and S2_sm are calculated as feature values, respectively, and representative segmental standard deviations S0_ms, S1_ms, S2_ms, S0_ss, S1_ss, and S2_ss are calculated as feature values, respectively.

From each segment's segmental mean values AS0_m, AS1_m and AS2_m and segmental standard deviations AS0_s, AS1_s and AS2_s, representative segmental mean values AS0_mm, AS1_mm, AS2_mm, AS0_sm, AS1_sm, and AS2_sm are calculated as feature values, respectively, and representative segmental standard deviations AS0_ms, AS1_ms, AS2_ms, AS0_ss, AS1_ss, and AS2_ss are calculated as feature values, respectively.

Stereognostic ability evaluation system 100 evaluates subject Sb1's stereognostic ability using at least one of a feature value derived from a value obtained by differentiating an inter-vehicle distance with time at least once (i.e., a first feature value) and a feature value derived from a slide amplitude (i.e., a second feature value). That is, the evaluation employs at least one of the feature values included in a region Rg in Fig. 8 surrounded by a dotted line.

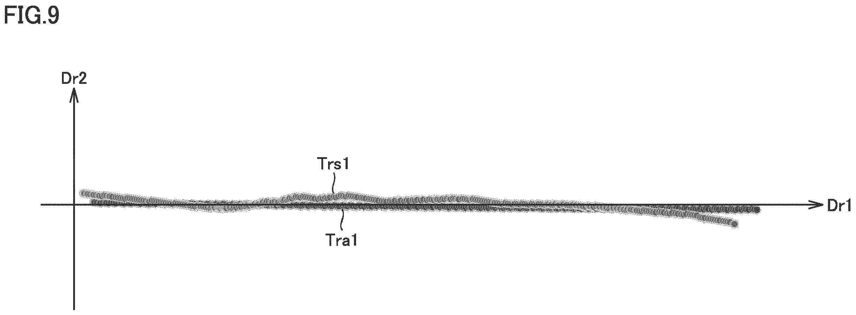

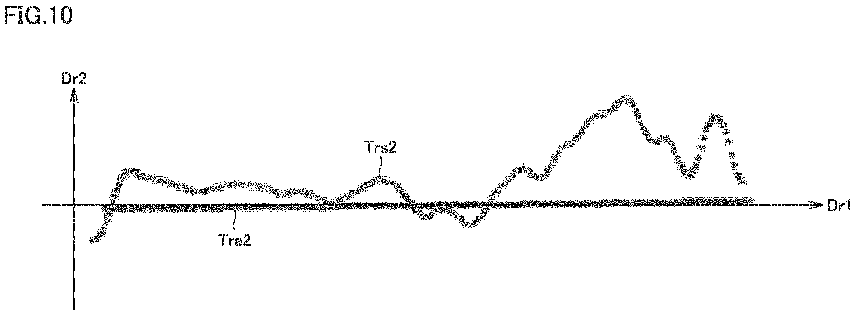

Hereinafter, reference will be made to Figs. 9 and 10 to describe a relationship between slide amplitude and stereognostic ability. Fig. 9 is a diagram representing together a traj ectory Tra1 of preceding automobile Ca1 and a traj ectory Trs1 of automobile Cs1 in Fig. 5 when automobile Cs1 is driven by a driver provided with a label indicating that the driver has a high stereognostic ability. Fig. 10 is a diagram representing together a trajectory Tra2 of preceding automobile Ca1 and a trajectory Trs2 of automobile Cs1 in Fig. 5 when automobile Cs1 is driven by a driver provided with a label indicating that the driver has a low stereognostic ability. As shown in Fig. 9, trajectory Trs1 substantially follows trajectory Tra1. On the other hand, as shown in Fig. 10, trajectory Trs2 often moves away from trajectory Tra2 and meanders. The driver having a relatively low stereognostic ability tends to have a lower ability to maintain the direction in which the automobile travels than the driver having a relatively high stereognostic ability. Therefore, a feature value derived from a slide amplitude is effective for evaluating the stereognostic ability of the driver (or a subject) of the automobile of interest as a feature value reflecting an ability to maintain the direction in which the automobile travels.

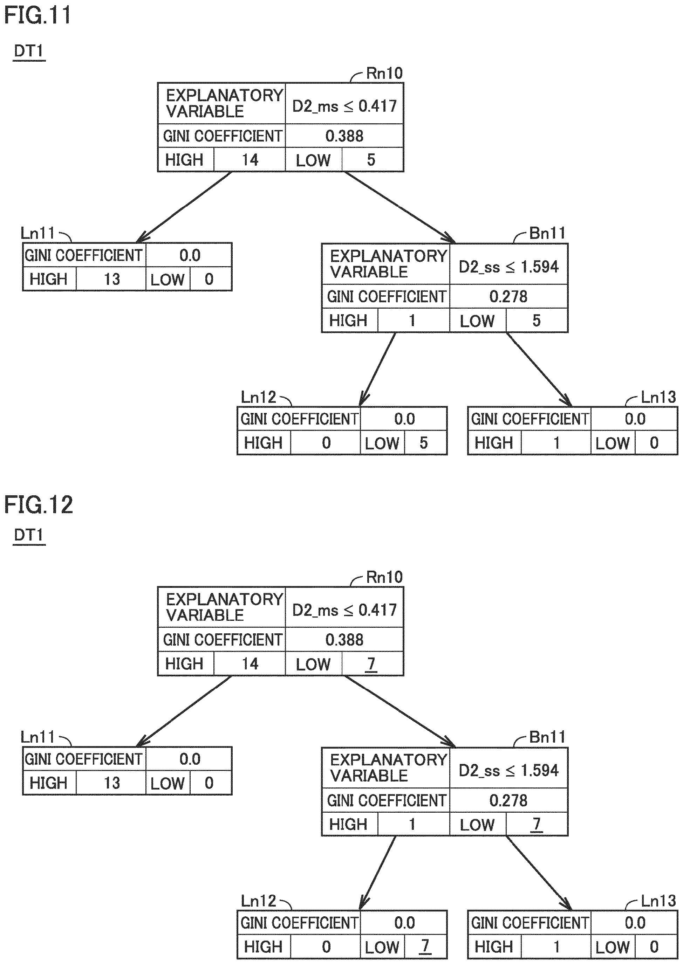

A specific example of decision tree DT of Fig. 2 will be described below with reference to Figs. 11 to 17. Fig. 11 is a diagram of an example of decision tree DT of Fig. 2, showing a decision tree DT1 including a feature value derived from interval acceleration D2 as an explanatory variable. As shown in Fig. 11, decision tree DT1 includes a root node Rn10, a branch node Bn11, and leaf nodes Ln11, Ln12, and Ln13. Of 19 drivers (or traveling data as training data) to be classified at root node Rn10, 14 drivers are provided with a label indicating a stereognostic ability (or an objective variable) of a high level ("high") and 5 drivers are provided with a label indicating a stereognostic ability of a low level ("low").

Root node Rn10 includes feature value D2_ms (a representative segmental standard deviation of segmental mean values of interval acceleration D2) as an explanatory variable. When a driver has feature value D2_ms of 0.417 or less, the driver is classified to leaf node Ln11. When a driver has feature value D2_ms larger than 0.417, the driver is classified to branch node Bn11. Of the 19 drivers to be classified at root node Rn10, 13 drivers are classified to leaf node Ln11, and 6 drivers are classified to branch node Bn11. Any driver classified to leaf node Ln11 is labeled "high." Of the 6 drivers classified to branch node Bn11, one driver is labeled "high" and 5 drivers are labeled "low."

Branch node Bn11 includes feature value D2_ss (a representative segmental standard deviation of segmental standard deviations of interval acceleration D2) as an explanatory variable. When a driver has feature value D2_ss of 1.594 or less, the driver is classified to leaf node Ln12. When a driver has feature value D2_ss larger than 1.594, the driver is classified to leaf node Ln13. Of the 6 drivers to be classified at branch node Bn11, 5 drivers are classified to leaf node Ln12, and one driver is classified to leaf node Ln13. Any driver classified to leaf node Ln12 is labeled "low." Any driver classified to leaf node Ln13 is labeled "high."

As described above, decision tree DT1 includes feature values D2_ms and D2_ss as explanatory variables. Decision tree DT1 classifies subjects with high stereognostic abilities to leaf nodes Ln11 and Ln13, and classifies subjects with a low stereognostic ability to leaf node Ln12.

A Gini coefficient indicated at each node of decision tree DT1 is an index value indicating to what extent a plurality of types are mixed in labels provided to drivers corresponding to the node (i.e., impurity). A larger Gini coefficient indicates that the plurality of types are mixed in the labels provided to the drivers to a larger extent. For example, the labels provided to the drivers classified to each of leaf nodes Ln11 to Ln13 are one type of label and no other label is not mixed, and accordingly, the leaf nodes' Gini coefficients will be a minimum value of 0. On the other hand, for example, the labels provided to the drivers classified to branch node Bn11 include labels "high" and "low" mixed together, and accordingly, the leaf node's Gini coefficient is larger than 0.

Fig. 12 is a diagram showing a classification result when a driver having a low stereognostic ability is added to be classified according to decision tree DT1 of Fig. 11. As shown in Fig. 12, 2 drivers with a label "low" are added to be classified at root node Rn10 shown in Fig. 11. The drivers are classified from root node Rn10 through branch node Bn11 to leaf node Ln12, similarly as has been described with reference to Fig. 11. Since decision tree DT1 has versatility for traveling data other than training data, it can determine a stereognostic ability of a subject with high accuracy.

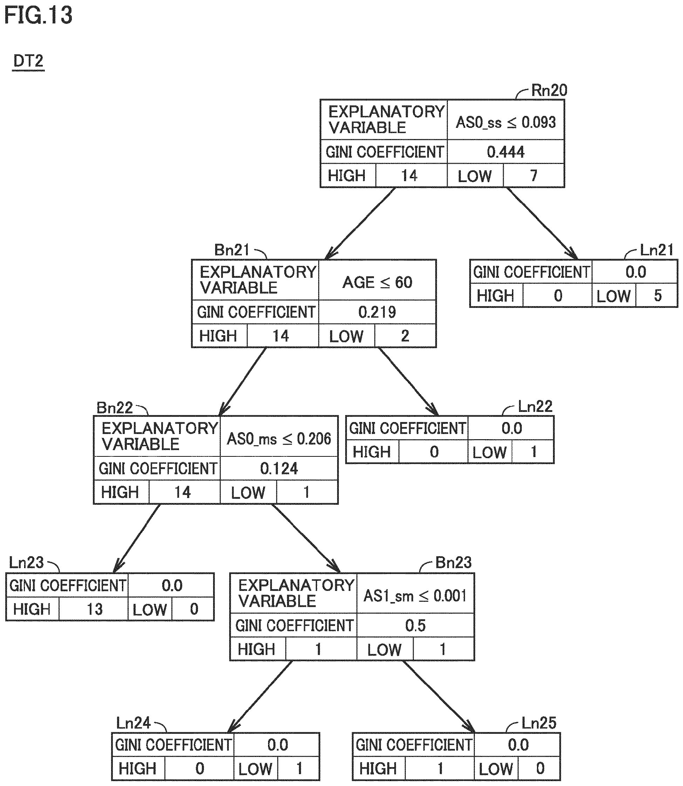

Fig. 13 is a diagram of another example of decision tree DT of Fig. 2, showing a decision tree DT2 including, as explanatory variables, feature values derived from slide amplitude S0 in absolute value AS0 and slide velocity S1 in absolute value AS1. As shown in Fig. 13, decision tree DT2 includes a root node Rn20, branch nodes Bn21, Bn22 and Bn23, and leaf nodes Ln21, Ln22, Ln23, Ln24 and Ln25. Of 21 drivers to be classified at root node Rn20, 14 drivers are labeled "high" and 7 drivers are labeled "low."

Root node Rn20 includes feature value AS0_ss (a representative segmental standard deviation of segmental standard deviations of slide amplitude S0 in absolute value AS0) as an explanatory variable. When a driver has feature value AS0_ss of 0.093 or less, the driver is classified to branch node Bn21. When a driver has feature value AS0_ss larger than 0.093, the driver is classified to leaf node Ln21. Of 21 drivers to be classified at root node Rn20, 16 drivers are classified to branch node Bn21, and 5 drivers are classified to leaf node Ln21. Any driver classified to leaf node Ln21 is labeled "low." Of the 16 drivers classified to branch node Bn21, 14 drivers are labeled "high" and 2 drivers are labeled "low."

Branch node Bn21 includes age as an explanatory variable. When a driver's age is 60 years or lower, the driver is classified to branch node Bn22. When a driver's age is higher than 60 years, the driver is classified to leaf node Ln22. Of the 16 drivers to be classified at branch node Bn21, 15 drivers are classified to branch node Bn22, and one driver is classified to leaf node Ln22. Any driver classified to leaf node Ln22 is labeled "low." Of the 15 drivers classified to branch node Bn22, 14 drivers are labeled "high" and one driver is labeled "low."

Branch node Bn22 includes, as an explanatory variable, feature value AS0_ms (a representative segmental standard deviation of segmental mean values of slide amplitude S0 in absolute value AS0). When a driver has feature value AS0_ms of 0.206 or less, the driver is classified to leaf node Ln23. When a driver has feature value AS0_ms larger than 0.206, the driver is classified to branch node Bn23. Of the 15 drivers to be classified at branch node Bn22, 13 drivers are classified to leaf node Ln23, and 2 drivers are classified to branch node Bn23. Any driver classified to leaf node Ln23 is labeled "high." Of the 2 drivers classified to branch node Bn23, one driver is labeled "high" and one driver is labeled "low."

Branch node Bn23 includes, as an explanatory variable, feature value AS1_sm (a representative segmental mean value of segmental standard deviations of slide velocity S1 in absolute value AS1). When a driver has feature value AS1_sm of 0.001 or less, the driver is classified to leaf node Ln24. When a driver has feature value AS1_sm larger than 0.001, the driver is classified to leaf node Ln25. Of the 2 drivers to be classified at branch node Bn23, one driver is classified to leaf node Ln24, and one driver is classified to branch node Bn25. Any driver classified to leaf node Ln24 is labeled "low." Any driver classified to leaf node Ln25 is labeled "low."

As described above, decision tree DT2 includes feature values AS0_ss, AS0_ms, AS1_sm, and age as explanatory variables. Decision tree DT2 classifies subjects with high stereognostic abilities to leaf nodes Ln23 and Ln25, and classifies subjects with low stereognostic abilities to leaf nodes Ln21, Ln22 and Ln24.

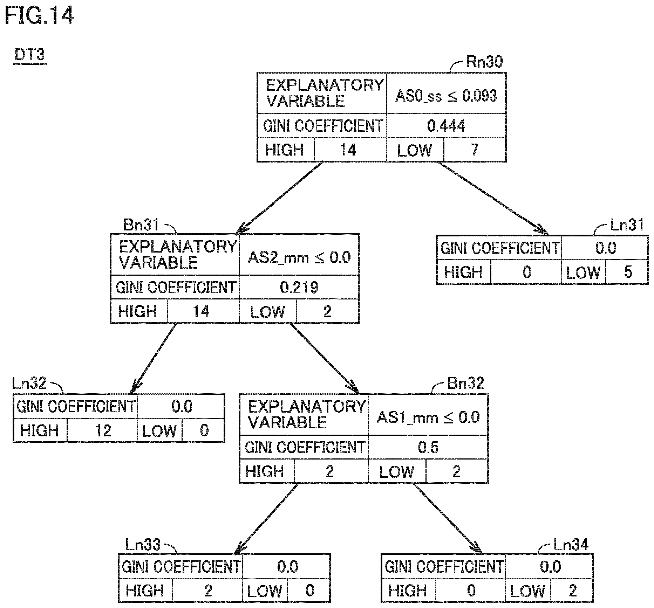

Fig. 14 is a diagram of another example of decision tree DT of Fig. 2, showing a decision tree DT3 including, as explanatory variables, feature values derived from slide amplitude S0 in absolute value AS0, slide velocity S1 in absolute value AS1, and slide acceleration S2 in absolute value AS2. As shown in Fig. 14, decision tree DT3 includes a root node Rn30, branch nodes Bn31 and Bn32, and leaf nodes Ln31, Ln32, Ln33 and Ln34. Of 21 drivers to be classified at root node Rn30, 14 drivers are labeled "high" and 7 drivers are labeled "low."

Root node Rn30 includes feature value AS0_ss (a representative segmental standard deviation of segmental standard deviations of slide amplitude S0 in absolute value AS0) as an explanatory variable. When a driver has feature value AS0_ss of 0.093 or less, the driver is classified to branch node Bn31. When a driver has feature value AS0_ss larger than 0.093, the driver is classified to leaf node Ln31. Of the 21 drivers to be classified at root node Rn30, 16 drivers are classified to branch node Bn31, and 5 drivers are classified to leaf node Ln31. Any driver classified to leaf node Ln31 is labeled "low." Of the 16 drivers classified to branch node Bn31, 14 drivers are labeled "high" and 2 drivers are labeled "low."

Branch node Bn31 includes, as an explanatory variable, feature value AS2_mm (a representative segmental mean value of segmental mean values of slide acceleration S2 in absolute value AS2). When a driver has feature value AS2_mm of 0.0 or less, the driver is classified to leaf node Ln32. When a driver has feature value AS2_mm larger than 0.0, the driver is classified to branch node Bn32. Of the 16 drivers to be classified at branch node Bn31, 12 drivers are classified to leaf node Ln32, and 4 drivers are classified to branch node Bn32. Any driver classified to leaf node Ln32 is labeled "high." Of the 4 drivers classified to branch node Bn32, 2 drivers are labeled "high" and 2 drivers are labeled "low."

Branch node Bn33 includes, as an explanatory variable, feature value AS1_mm (a representative segmental mean value of segmental mean values of slide velocity S1 in absolute value AS1). When a driver has feature value AS1_mm of 0.0 or less, the driver is classified to leaf node Ln33. When a driver has feature value AS1_mm larger than 0.0, the driver is classified to leaf node Ln34. Of the 4 drivers to be classified at branch node Bn32, 2 drivers are classified to leaf node Ln33, and 2 drivers are classified to leaf node Ln34. Any driver classified to leaf node Ln33 is labeled "high." Any driver classified to leaf node Ln34 is labeled "low."

As described above, decision tree DT3 includes feature values AS0_ss, AS2_mm, and AS1_mm as explanatory variables. Decision tree DT3 classifies subjects with high stereognostic abilities to leaf nodes Ln32 and Ln33, and classifies subjects with low stereognostic abilities to leaf nodes Ln31 and Ln34.

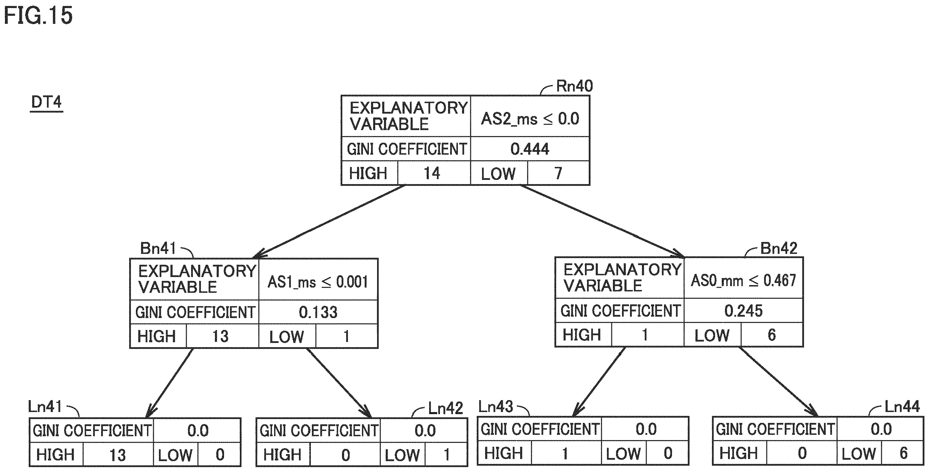

Fig. 15 is a diagram of another example of decision tree DT of Fig. 2, showing a decision tree DT4 including, as explanatory variables, feature values derived from slide amplitude S0 in absolute value AS0, slide velocity S1 in absolute value AS1, and slide acceleration S2 in absolute value AS2. As shown in Fig. 15, decision tree DT4 includes a root node Rn40, branch nodes Bn41 and Bn42, and leaf nodes Ln41, Ln42, Ln43 and Ln44. Of 21 drivers to be classified at root node Rn40, 14 drivers are labeled "high" and 7 drivers are labeled "low."

Root node Rn40 includes, as an explanatory variable, feature value AS2_ms (a representative segmental standard deviation of segmental mean values of slide acceleration S2 in absolute value AS2). When a driver has feature value AS2_ms of 0.0 or less, the driver is classified to branch node Bn41. When a driver has feature value AS2_ms larger than 0.0, the driver is classified to branch node Bn42. Of the 21 drivers to be classified at root node Rn40, 14 drivers are classified to branch node Bn41, and 7 drivers are classified to branch node Bn42. Of the 14 drivers classified to branch node Bn41, 13 drivers are labeled "high" and one driver is labeled "low." Of the 7 drivers classified to branch node Bn42, one driver is labeled "high" and 6 drivers are labeled "low."

Branch node Bn41 includes, as an explanatory variable, feature value AS1_ms (a representative segmental standard deviation of segmental mean values of slide velocity S1 in absolute value AS1). When a driver has feature value AS1_ms of 0.001 or less, the driver is classified to leaf node Ln41. When a driver has feature value AS1_ms larger than 0.001, the driver is classified to leaf node Ln42. Any driver classified to leaf node Ln41 is labeled "high." Any driver classified to leaf node Ln42 is labeled "low."

Branch node Bn42 includes, as an explanatory variable, feature value AS0_mm (a representative segmental mean value of segmental mean values of slide amplitude S0 in absolute value AS0). When a driver has feature value AS0_mm of 0.467 or less, the driver is classified to leaf node Ln43. When a driver has feature value AS0_mm larger than 0.467, the driver is classified to leaf node Ln44. Any driver classified to leaf node Ln43 is labeled "high." Any driver classified to leaf node Ln44 is labeled "low."

As described above, decision tree DT4 includes feature values AS2_ms, AS1_ms, and AS0_mm as explanatory variables. Decision tree DT4 classifies subjects with high stereognostic abilities to leaf nodes Ln41 and Ln43, and classifies subjects with low stereognostic abilities to leaf nodes Ln42 and Ln44.

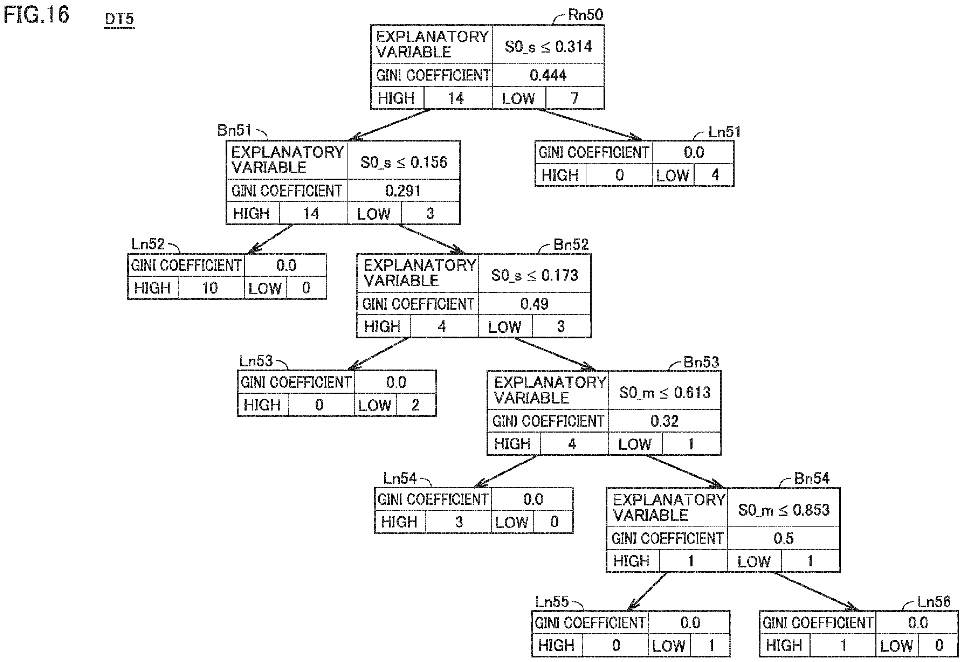

Fig. 16 is a diagram of another example of decision tree DT of Fig. 2, showing a decision tree DT5 including a feature value derived from slide amplitude S0 as an explanatory variable. As shown in Fig. 16, decision tree DT5 includes a root node Rn50, branch nodes Bn51, Bn52, Bn53 and Bn54, and leaf nodes Ln51, Ln52, Ln53, Ln54, Ln55 and Ln56. Of 21 drivers to be classified at root node Rn50, 14 drivers are labeled "high" and 7 drivers are labeled "low."

Root node Rn50 includes, as an explanatory variable, feature value S0_s (a segmental standard deviation of slide amplitude S0). When a driver has feature value S0_s of 0.314 or less, the driver is classified to branch node Bn51. When a driver has feature value S0_s larger than 0.314, the driver is classified to leaf node Ln51. Of the 21 drivers to be classified at root node Rn50, 17 drivers are classified to branch node Bn51, and 4 drivers are classified to leaf node Ln51. Any driver classified to leaf node Ln51 is labeled "low." Of the 17 drivers classified to branch node Bn51, 14 drivers are labeled "high" and 3 drivers are labeled "low."

Branch node Bn51 includes feature value S0_s as an explanatory variable. When a driver has feature value S0_s of 0.156 or less, the driver is classified to leaf node Ln52. When a driver has feature value S0_s larger than 0.156, the driver is classified to branch node Bn52. Of the 17 drivers to be classified at branch node Bn51, 10 drivers are classified to leaf node Ln52, and 7 drivers are classified to branch node Bn52. Any driver classified to leaf node Ln52 is labeled "high." Of the 7 drivers classified to branch node Bn52, 4 drivers are labeled "high" and 3 drivers are labeled "low."

Branch node Bn52 includes feature value S0_s as an explanatory variable. When a driver has feature value S0_s of 0.173 or less, the driver is classified to leaf node Ln53. When a driver has feature value S0_s larger than 0.173, the driver is classified to branch node Bn53. Of the 7 drivers to be classified at branch node Bn52, 2 drivers are classified to leaf node Ln53, and 5 drivers are classified to branch node Bn53. Any driver classified to leaf node Ln53 is labeled "low." Of the 5 drivers classified to branch node Bn53, 4 drivers are labeled "high" and one driver is labeled "low."

Branch node Bn53 includes feature value S0_m as an explanatory variable. When a driver has feature value S0_m of 0.613 or less, the driver is classified to leaf node Ln54. When a driver has feature value S0_m larger than 0.613, the driver is classified to branch node Bn54. Of the 5 drivers to be classified at branch node Bn53, 3 drivers are classified to leaf node Ln54, and 2 drivers are classified to branch node Bn54. Any driver classified to leaf node Ln54 is labeled "high." Of the 2 drivers classified to branch node Bn54, one driver is labeled "high" and one driver is labeled "low."

Branch node Bn54 includes feature value S0_m as an explanatory variable. When a driver has feature value S0_m of 0.853 or less, the driver is classified to leaf node Ln55. When a driver has feature value S0_m larger than 0.853, the driver is classified to leaf node Ln56. Of the 2 drivers to be classified at branch node Bn54, one driver is classified to leaf node Ln55, and one driver is classified to leaf node Ln56. Any driver classified to leaf node Ln55 is labeled "low." Any driver classified to leaf node Ln56 is labeled "high."

As described above, decision tree DT5 includes feature values S0_s and S0_m as explanatory variables. Decision tree DT5 classifies subjects with high stereognostic abilities to leaf nodes Ln52, Ln54, and Ln56, and classifies subjects with low stereognostic abilities to leaf nodes Ln51, Ln53, and Ln55.

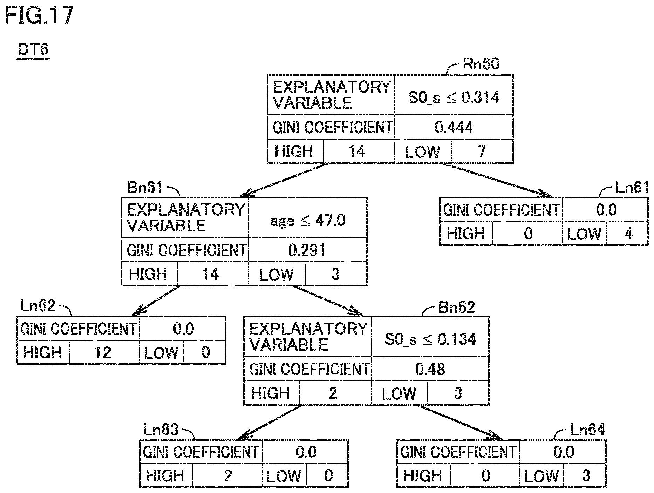

Fig. 17 is a diagram of another example of decision tree DT of Fig. 2, showing a decision tree DT6 including a feature value derived from slide amplitude S0 as an explanatory variable. As shown in Fig. 17, decision tree DT6 includes a root node Rn60, branch nodes Bn61 and Bn62, and leaf nodes Ln61, Ln62, Ln63 and Ln64. Of 21 drivers to be classified at root node Rn60, 14 drivers are labeled "high" and 7 drivers are labeled "low."

Root node Rn60 includes feature value S0_s as an explanatory variable. When a driver has feature value S0_s of 0.314 or less, the driver is classified to branch node Bn61. When a driver has feature value S0_s larger than 0.314, the driver is classified to leaf node Ln61. Of the 21 drivers to be classified at root node Rn60, 17 drivers are classified to branch node Bn61, and 4 drivers are classified to leaf node Ln61. Any driver classified to leaf node Ln61 is labeled "low." Of the 17 drivers classified to branch node Bn61, 14 drivers are labeled "high" and 3 drivers are labeled "low."

Branch node Bn61 includes age as an explanatory variable. When a driver's age is 47 years or younger, the driver is classified to leaf node Ln62. If a driver's age is older than 47 years, the driver is classified to branch node Bn62. Of the 17 drivers to be classified at branch node Bn61, 12 drivers are classified to leaf node Ln62, and 5 drivers are classified to branch node Bn62. Any driver classified to leaf node Ln62 is labeled "high." Of the 5 drivers classified to branch node Bn62, 2 drivers are labeled "high" and 3 drivers are labeled "low."

Branch node Bn62 includes feature value S0_s as an explanatory variable. When a driver has feature value S0_s of 0.134 or less, the driver is classified to leaf node Ln63. When a driver has feature value S0_s larger than 0.134, the driver is classified to leaf node Ln64. Of the 5 drivers to be classified at branch node Bn62, 2 drivers are classified to leaf node Ln63, and 3 drivers are classified to leaf node Ln64. Any driver classified to leaf node Ln63 is labeled "high." Any driver classified to leaf node Ln64 is labeled "low."

As described above, decision tree DT6 includes feature value S0_s and age as explanatory variables. Decision tree DT6 classifies subjects with high stereognostic abilities to leaf nodes Ln62 and Ln63, and classifies subjects with low stereognostic abilities to leaf nodes Ln61 and Ln64.

When decision tree DT5 of Fig. 16 and decision tree DT6 of Fig. 17 are compared, they are identical for a condition for an explanatory variable included in a root node and a target to be classified. In contrast, for an explanatory variable, decision tree DT5 does not have an explanatory variable including information about an attribute of a subject, whereas decision tree DT6 has an explanatory variable including information about an attribute of a subject. As a result, with respect to how many times the decision trees branch (with respect to the root node and the number of branch nodes), decision tree DT5 branches five times, whereas decision tree DT6 branches three times. Thus, an explanatory variable including information about an attribute of a subject allows a decision tree to be generated to have an efficient branch structure.

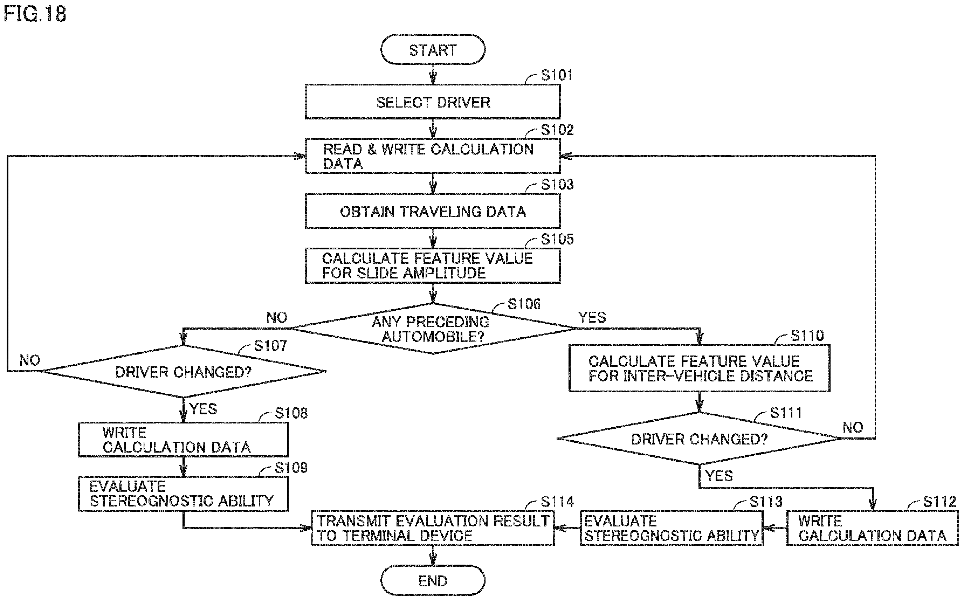

Fig. 18 is a flowchart of a process in the stereognostic ability evaluation method performed by processor 101 executing stereognostic ability evaluation program 103a of Fig. 2. Hereinafter, a step will simply be denoted as S.

As shown in Fig. 18, in S101, processor 101 selects subject Sb1 as a driver for automobile Cs1, and proceeds to S102. In S102, processor 101 reads calculation data (for example, a feature value derived from previous traveling data) for subject Sb1 from storage 103, and proceeds to S103. In S103, processor 101 acquires the current traveling data from drive recorder 900, and proceeds to S105. In S105, processor 101 calculates a feature value for a slide amplitude, and proceeds to S106. In S106, processor 101 determines whether any preceding automobile is present.

When there is no preceding automobile (NO in S106), processor 101 determines whether the driver is changed in S107. When the driver is not changed (NO in S107), processor 101 returns to S102, and writes to storage 103 calculation data including the feature value and/or the like calculated in S105. When the driver is changed (YES in S107), then, in S108, processor 101 writes to storage 103 the calculation data including the feature value and/or the like calculated in S105, and proceeds to S109. In S109, processor 101 uses evaluation model 103c to evaluate subject Sb1's stereognostic ability based on the feature value for the slide amplitude, and proceeds to S114.

When there is a preceding automobile (YES in S106), processor 101 calculates a feature value for an inter-vehicle distance in S110, and proceeds to S111. Processor 101 determines whether the driver is changed in S111. When the driver is not changed (NO in 5111), processor 101 returns to S102, and writes to storage 103 calculation data including the feature values calculated in S105 and S110. When the driver is changed (YES in S111), then, in S112, processor 101 writes to storage 103 the calculation data including the feature values calculated in S105 and S 110, and proceeds to S113. In S113, processor 101 uses evaluation model 103c to evaluate subject Sb1's stereognostic ability based on the feature values for the slide amplitude and the inter-vehicle distance, and proceeds to S114. In S114, processor 101 transmits an evaluation result of the stereognostic ability to the terminal device of subject Sb1, and ends the process.

According to stereognostic ability evaluation system 100, evaluation model 103c trained by machine learning can be used to objectively evaluate a subject's stereognostic ability based on a quantified feature value (see Fig. 8).

### [Exemplary Variation of First Embodiment]

In the first embodiment has been described a configuration in which information processing device 110 has both a function to evaluate stereognostic ability and a function to train an evaluation model to be a trained evaluation model. In an exemplary variation of the first embodiment will be described a configuration in which a device has the function to evaluate stereognostic ability and another device has the training function.

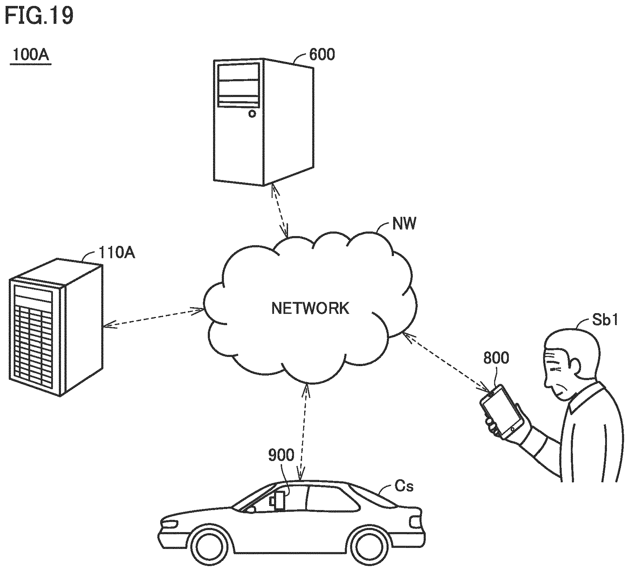

Fig. 19 is a block diagram showing a configuration of a stereognostic ability evaluation system 100A according to an exemplary variation of the first embodiment. Stereognostic ability evaluation system 100A is configured such that information processing device 110 shown in Fig. 1 is replaced with an information processing device 110A and a training device 600 is added. Except for this, stereognostic ability evaluation system 100A has a configuration similar to that of stereognostic ability evaluation system 100 of Fig. 1, and accordingly, will not be described repeatedly for that configuration. As shown in Fig. 19, information processing device 110A, training device 600, terminal device 800, automobile Cs1, and drive recorder 900 are interconnected via network NW.

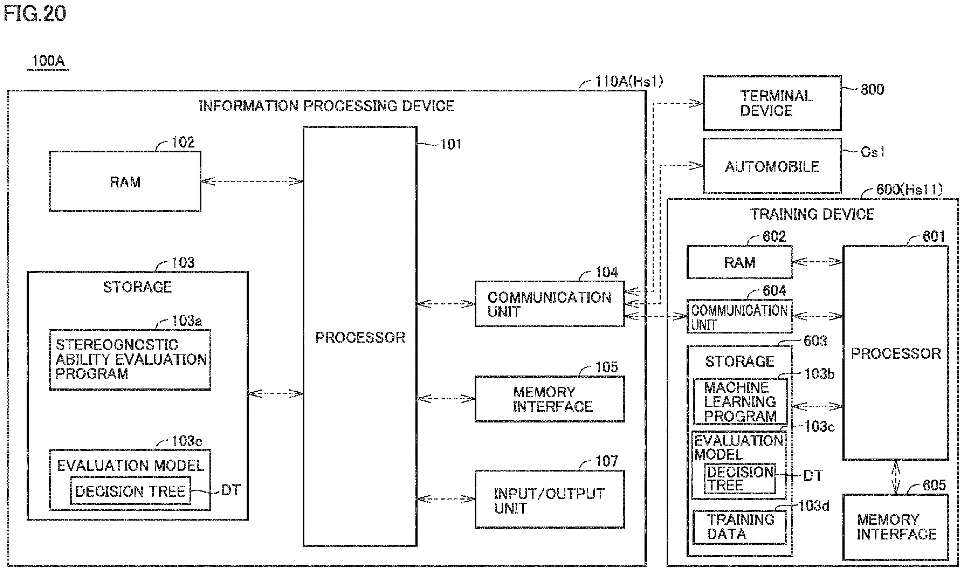

Fig. 20 is a diagram showing a hardware configuration of stereognostic ability evaluation system 100A of Fig. 19. Information processing device 110A has a hardware configuration, which is a configuration in which machine learning program 103b and training data 103d are removed from storage 103 in Fig. 2. Except for this, information processing device 110A has a hardware configuration similar to that of information processing device 110 shown in Fig. 2, and accordingly, will not be described repeatedly for that hardware configuration.

As shown in Fig. 20, training device 600 includes a processor 601, a RAM 602, a storage 603, a communication unit 604, a memory interface 605, and a housing Hs11. Housing Hs11 accommodates processor 601, RAM 602, storage 603, communication unit 604, and memory interface 605. Processor 601, RAM 602, storage 603, communication unit 604, and memory interface 605 have functions similar to those of the Fig. 2 processor 101, RAM 102, storage 103, communication unit 104, and memory interface 105, respectively, and accordingly, will not be described repeatedly for the functions.

Storage 603 stores machine learning program 103b, evaluation model 103c, and training data 103d. Processor 601 executes machine learning program 103b to train evaluation model 103c to be a trained evaluation model. Processor 601 provides the trained evaluation model 103c to information processing device 110A via communication unit 604.

Thus, the stereognostic ability evaluation system, stereognostic ability evaluation device, stereognostic ability evaluation program, and stereognostic ability evaluation method according to the first embodiment and an exemplary variation thereof can implement objective evaluation of stereognostic ability.

### [Second Embodiment]

In the first embodiment and an exemplary variation thereof has been described a configuration in which a subject's stereognostic ability is evaluated based on a positional change of a moving object operated by the subject in the real world. In a second embodiment will be described a configuration for evaluating a subject's stereognostic ability based on a positional change of a moving object operated by the subject in a virtual reality (or a virtual space). While, as well as the first embodiment, the second embodiment will also be described with a moving object as an automobile, the moving object is not limited to an automobile. Any moving object may be used insofar as it is positionally changed in response to an operation by the subject, and the moving object may for example be a motorcycle, a bicycle, an airplane, or a ship.

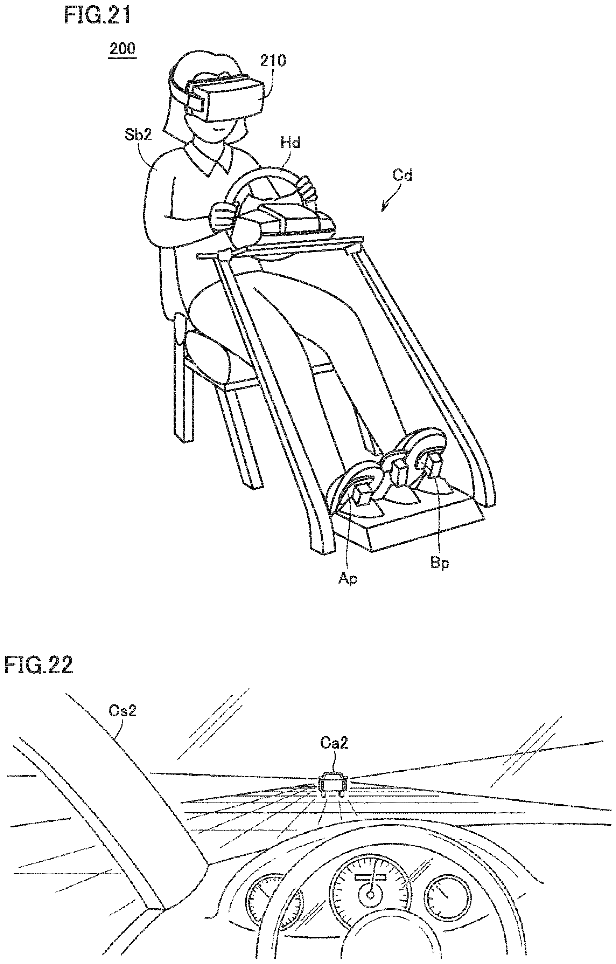

Fig. 21 is a diagram showing a state in which a subject Sb2 has his/her stereognostic ability measured in a measurement test through a drive simulation in a stereognostic ability evaluation system 200 according to the second embodiment. As shown in Fig. 21, stereognostic ability evaluation system 200 comprises a stereognostic ability evaluation device 210 and a control device Cd. Stereognostic ability evaluation device 210 and control device Cd are interconnected wirelessly or by wire. Control device Cd includes a handle Hd, an accelerator pedal Ap, and a brake pedal Bp. As well as the stereognostic ability evaluation system according to the first embodiment, stereognostic ability evaluation system 200 evaluates subject Sb2's stereognostic ability using at least one of a feature value derived from a value obtained by differentiating an inter-vehicle distance with time at least once and a feature value derived from a slide amplitude.

Fig. 22 is a diagram showing an example of a screen displayed for subject Sb2 via stereognostic ability evaluation device 210 in the drive simulation by stereognostic ability evaluation system 200 of Fig. 21. As shown in Fig. 22, for subject Sb2, a scenery is displayed as viewed at the driver's seat of an automobile Cs2 (or a moving object) driven by subject Sb2 in a virtual space. In the scenery, a preceding automobile Ca2 (or a reference object) traveling in front of automobile Cs2 is displayed. Automobile Cs2 moves in the virtual space in response to an input by subject Sb2 to the control device shown in Fig. 21. Note that preceding automobile Ca2 may not be displayed when subject Sb2's stereognostic ability is evaluated by using a feature value for a slide amplitude of automobile Cs2.

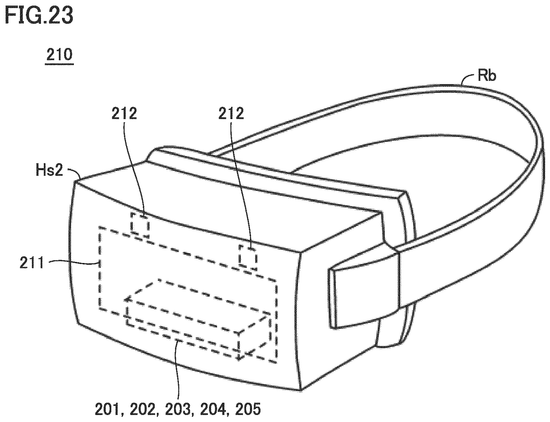

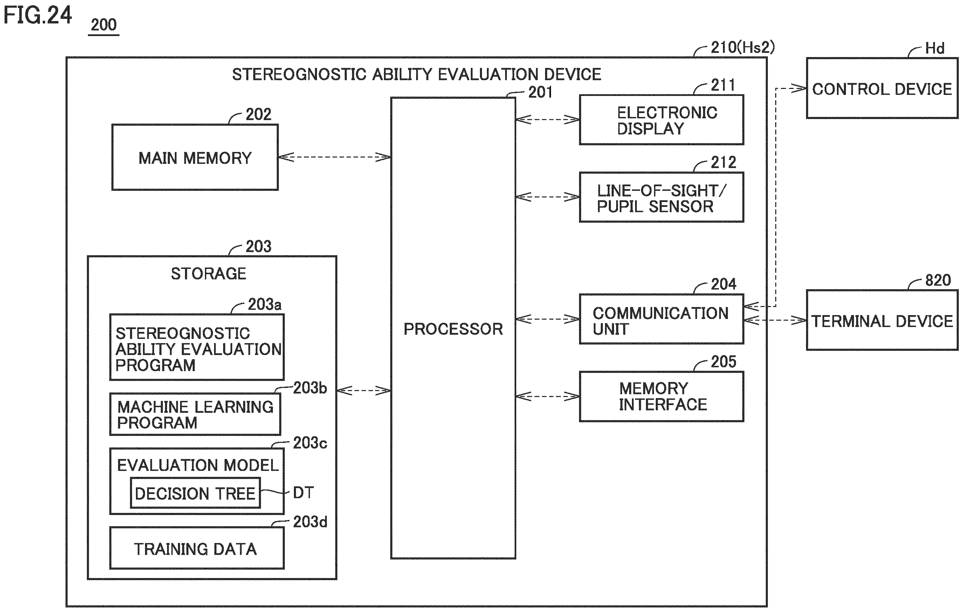

Fig. 23 is an external perspective view of stereognostic ability evaluation device 210 of Fig. 21. In Fig. 23, a configuration indicated by a broken line is accommodated in a housing Hs2 of stereognostic ability evaluation device 210 and externally invisible. The configuration will more specifically be described later with reference to Fig. 24. Stereognostic ability evaluation device 210 evaluates a subject's stereognostic ability by causing the subject to visually recognize a moving object and evaluating a response of the subject to the moving object.

Stereognostic ability evaluation device 210 is typically a head mounted display (or a goggle) comprising an electronic display that displays a video representing a three-dimensional virtual reality, and is in the form of a virtual reality headset. An attachment band Rb such as a rubber band is typically attached to stereognostic ability evaluation device 210. A user (a subject) wears stereognostic ability evaluation device 210 around his/her eyes by placing stereognostic ability evaluation device 210 so as to cover around the eyes and winding attachment band Rb on his/her head.

Fig. 24 is a diagram showing a hardware configuration of stereognostic ability evaluation device 210 of Fig. 23. As shown in Fig. 24, stereognostic ability evaluation device 210 includes a processor 201, a RAM 202, a storage 203, a communication unit 204, a memory interface 205, an electronic display 211, and a line-of-sight/pupil sensor 212. Processor 201, RAM 202, storage 203, communication unit 204, and memory interface 205 have functions similar to those of the Fig. 2 processor 101, RAM 102, storage 103, communication unit 104, and memory interface 105, respectively, and accordingly, will not be described repeatedly for the functions.

Storage 203 stores an OS (an operating system) a program (not shown), a stereognostic ability evaluation program 203a, a machine learning program 203b, an evaluation model 203c (a specific model), and training data 203d.

Electronic display 211 includes, for example, a flat panel display such as a liquid crystal display (LCD) or an organic electro luminescence (EL) display. Electronic display 211 displays a video of a moving object moving in a virtual space for a subject wearing stereognostic ability evaluation device 210 around the eyes via an eyepiece disposed on the subject's side. When the data of the video is transferred from processor 201 to a data buffer area of electronic display 211, electronic display 211 reads data of image from the data buffer area and displays the video represented by the data. Electronic display 211 has an electronic display for the right eye and an electronic display for the left eye independently, and the user sees them through their respective eyepieces. When the position of an object displayed on electronic display 211 is at infinity, the object is displayed at the same position on electronic displays 211 for the right and left eyes. As a result, no parallax is caused between the right and left eyes, and the right and left eyes are diverged, whereby a sense of presence at infinity can be given to the subject. As the position of the object approaches the user, the object is displayed inward on electronic display 211 for the right eye and electronic display 211 for the left eye. As a result, a parallax is caused between the right and left eyes, and the right and left eyes are converged, whereby a sense of presence close to the subject can be given to the subject.

Line-of-sight/pupil sensor 212 is disposed on an upper side of electronic display 211 toward the subject and senses the direction of the line of sight of each of the right and left eyes as well as the pupillary size of each eye. Line-of-sight/pupil sensor 212 acquires images of the right and left eyes by an image acquisition means such as a camera, determines a pupillary position and size in the images to determine a direction of a line of sight and a pupillary size, and outputs them as line-of-sight information to processor 201. The camera can be a visible light camera or an infrared camera. In order to determine that the subject visually recognizes the moving object, first of all, the direction of a line of sight is important data. By confirming that the lines of sight of the right and left eyes (or normals to the centers of the pupils) each exactly passes through the moving object, it can be confirmed that the subject visually recognizes the moving object. In doing so, when the subject visually recognizes a nearby moving object, the subject has his/her right and left eyes with their lines of sight turned inward or converged due to parallax. Further, a pupillary diameter can be additionally used in order to determine that the subject is continuously visually recognizing a moving object approaching the subject. When the subject is continuously visually recognizing a moving object approaching the subject, the subject's pupils become gradually smaller in diameter due to near pupillary reflex, and by detecting it, it can be confirmed whether the subject visually recognizes the moving object.

Stereognostic ability is predicated on a normal function of an eye (e.g., pupillary accommodation or eye movement) for acquisition of visual information. Therefore, a stereognostic ability is defined for example as an ability of a subject who observes a moving object to cerebrally, accurately understand a positional relationship of the moving object from visual information of the moving object and perform an operation corresponding to the moving object appropriately and accurately based on the understood positional relationship.

As a method for confirming a normal function of an eye, for example, pupillometry and near point measurement are known. These measurements can be done using an instrument referred to as TriIRIS. Pupillometry includes at least measuring a pupillary response to visible-light stimulation from a visual target (or a photopupillary response) and measuring a pupillary change when viewing a moving visual target. Specifically, a pupillary change is caused by near pupillary reflex, and pupillary constriction occurs as the visual target moves closer. In near point measurement, specifically, a subject presses a handheld switch when a visual target approaching at constant refraction rate is blurred during observation, and the position of the visual target at that time is recorded as a near point. These measurements measure a near point by a state of an eyeball (or pupil) and pressing of a switch when a visual target blurs and a main purpose thereof is to measure a near point.

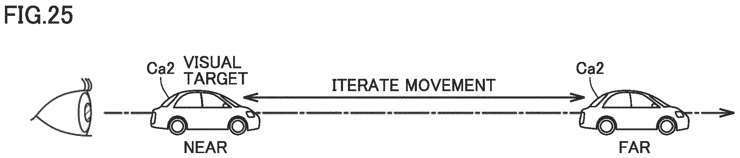

A stereognostic ability also includes an ability to ocularly, accurately track a moving object, correctly recognize its position, and accurately respond thereto, in particular. In order to measure a function to ocularly track a moving object, measuring pupillary accommodation and convergence response is considered particularly useful. These measurement methods will now be described below. Fig. 25 is a diagram representing a concept of a visual target used for measuring a function of eyes. The visual target (or preceding automobile Ca2) moves between a direction in which it moves away from the subject's eyes and a direction in which it approaches the subject's eyes such that it is visually recognized by the subject in front of his/her eyes. It is desirable to check the state of the eyes of the subject whenever the visual target is moved back and forth.

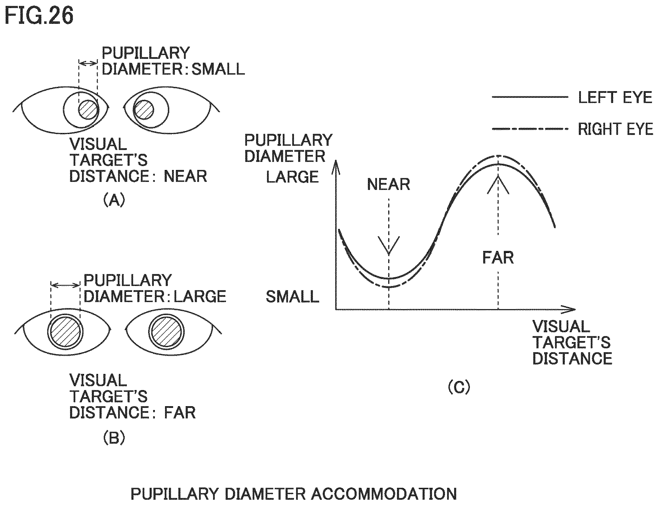

Fig. 26 is a diagram representing a relationship between a visual target's distance (a distance between an eye of a subject and the visual target) and pupillary diameter through near pupillary reflex. Fig. 26(A) shows that pupillary diameter decreases when the visual target's distance is short. Fig. 26(B) shows that pupillary diameter increases when the visual target's distance is long. Fig. 26(C) is a graph when the horizontal axis represents the visual target's distance and the vertical axis represents pupillary diameter. A solid line represents a graph of the left eye, and a dot-and-dash line represents a graph of the right eye. These graphs show that pupillary diameter decreases when the visual target's distance is short, and pupillary diameter increases when the visual target's distance is long. It is also shown that the right eye and the left eye have substantially the same pupillary diameter regardless of the visual target's distance.

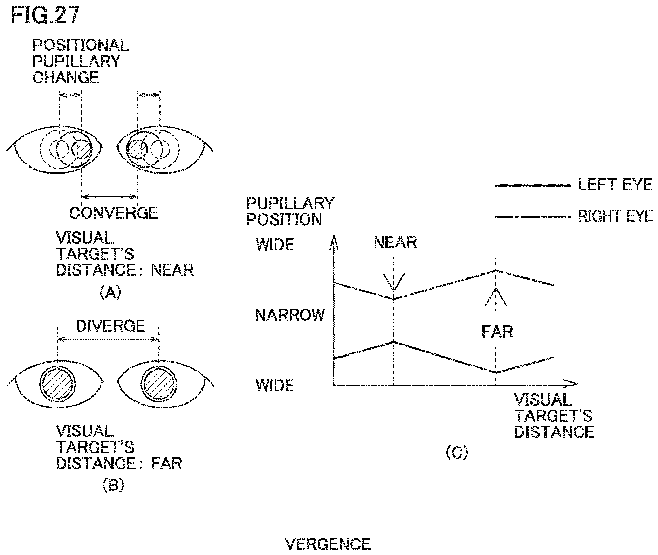

Fig. 27 is a diagram representing a relationship between a visual target's distance and pupillary position (or vergence). Fig. 27(A) shows that the right and left eyes turned inward or converged when the visual target's distance is short. Fig. 27(B) shows that the right and left eyes turned outward or diverged when the visual target's distance is long. Fig. 27(C) is a graph when the horizontal axis represents the visual target's distance and the vertical axis represents pupillary position. A solid line represents a graph of the left eye, and a dot-and-dash line represents a graph of the right eye. These graphs show that when the visual target's distance is short the right and left eyes have their pupils with a small distance therebetween and are thus converged, whereas when the visual target's distance is long the right and left eyes have their pupils with a large distance therebetween and are thus diverged.

When the visual target observed by the subject is moved to be near/far, the subject responds thereto by a pupillary change in diameter, vergence, and the like, as described above. If the subject has an impaired function for visual recognition, the subject is less responsive. Therefore, the subject's function for visual recognition can be measured by measuring the subject's pupillary change in diameter and vergence when the visual target is moved to be near/far. Stereognostic ability evaluation system 200 can dispense with a large-sized device to move a visual target to be near/far, and thus be miniaturized.

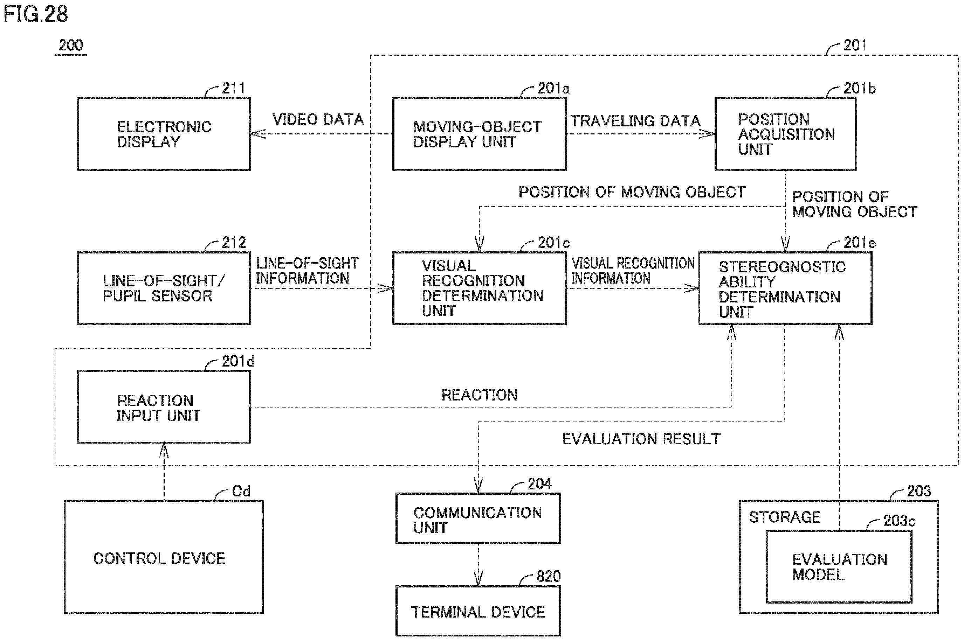

Fig. 28 is a functional block diagram showing a configuration of a function of processor 201 of Fig. 24 to evaluate stereognostic ability. In stereognostic ability evaluation system 200, processor 201 executes a stereognostic ability evaluation program 203a stored in storage 203 to configure a module forming functional blocks of a moving-object display unit 201a, a position acquisition unit 201b, a visual recognition determination unit 201c, a reaction input unit 201d, and a stereognostic ability determination unit 201e. Therefore, in place of processor 201 and stereognostic ability evaluation program 203a of Fig. 24, Fig. 28 shows functional blocks implemented thereby. These functional blocks will now be described below.

Moving-object display unit 201a continuously generates images of a scenery in front as viewed by subject Sb2 at the driver's seat of automobile Cs2 by three-dimensional rendering as automobile Cs2 proceeds, and transfers the images as video data to a data buffer area of electronic display 211. The video data is data for each of the right eye and the left eye for electronic display 211. Depending on the position of the moving object, the video data causes a parallax between the video data for the right eye and the video data for the left eye at a position in the scenery seen by subject Sb2 from automobile Cs2. Therefore, the subject who views the video data on electronic display 211 can view a ball with a realistic perspective. Moving-object display unit 201a transmits traveling data of automobile Cs2 based on the video data to position acquisition unit 201b.

Position acquisition unit 201b acquires traveling data of subject Sb2 from moving-object display unit 201a. Position acquisition unit 201b acquires information about a position of automobile Cs2 from the traveling data. Position acquisition unit 201b outputs the information about the position of automobile Cs2 to visual recognition determination unit 201c and stereognostic ability determination unit 201e.

Visual recognition determination unit 201c determines whether the subject's line of sight is in a direction correctly corresponding to the position of automobile Cs2 to determine whether subject Sb2 visually, spatially recognizes automobile Cs2. Visual recognition determination unit 201c receives the data of the directions of the lines of sight of the right and left eyes of subject Sb2 as sensed by line-of-sight/pupil sensor 212, and determines whether the directions of the lines of sight of the right and left eyes correspond to the position of automobile Cs2 as transmitted from moving-object display unit 201a to determine whether subject Sb2 spatially recognizes automobile Cs2. Visual recognition determination unit 201c may further receive data of the pupillary diameters of the right and left eyes of subject Sb2 as sensed by line-of-sight/pupil sensor 212. Visual recognition determination unit 201c may operate to determine that the subject spatially recognizes an object when it is further determined that the subject's both eyes have a gradually decreasing pupillary diameter while preceding automobile Ca2 positionally approaches a predetermined viewpoint and thus has a smaller distance to the subject (that is, when near pupillary reflex occurs in response to a reduced distance). Visual recognition determination unit 201c outputs the determination result as visual recognition information to stereognostic ability determination unit 201e.

Reaction input unit 201d receives an input of an active reaction made by subject Sb2 to correspond to a three-dimensional position of automobile Cs2 as recognized by subject Sb2 in a virtual space. Based on the information input by subject Sb2 to control device Cd, reaction input unit 201d determines the reaction of subject Sb2 driving automobile Cs2. Reaction input unit 201d outputs information about the reaction to stereognostic ability determination unit 201e.

Based on the information about the position of automobile Cs2, stereognostic ability determination unit 201e derives a feature value derived from at least one of an interval velocity of automobile Cs2, an interval acceleration thereof, a slide amplitude in absolute value thereof, a slide velocity in absolute value thereof, a slide acceleration in absolute value thereof, the slide amplitude, the slide velocity, and the slide acceleration. Stereognostic ability determination unit 201e uses evaluation model 203c to determine the stereognostic ability of subject Sb2 from the feature value and the visual recognition information received from visual recognition determination unit 201c. The visual recognition information may not be used to determine the stereognostic ability of subject Sb2. Further, the information received from reaction input unit 201d about the reaction may be used to determine the stereognostic ability of subject Sb2. Stereognostic ability determination unit 201e transmits an evaluation result of the stereognostic ability to a terminal device 820 of subject Sb2 via communication unit 204.

In stereognostic ability evaluation system 200, when machine learning program 203b is executed by processor 201, stereognostic ability evaluation device 210 functions as a training device to train evaluation model 203c to be a trained evaluation model. As well as in the exemplary variation of the first embodiment, a training device separate from stereognostic ability evaluation device 210 may subject evaluation model 203c to machine learning.

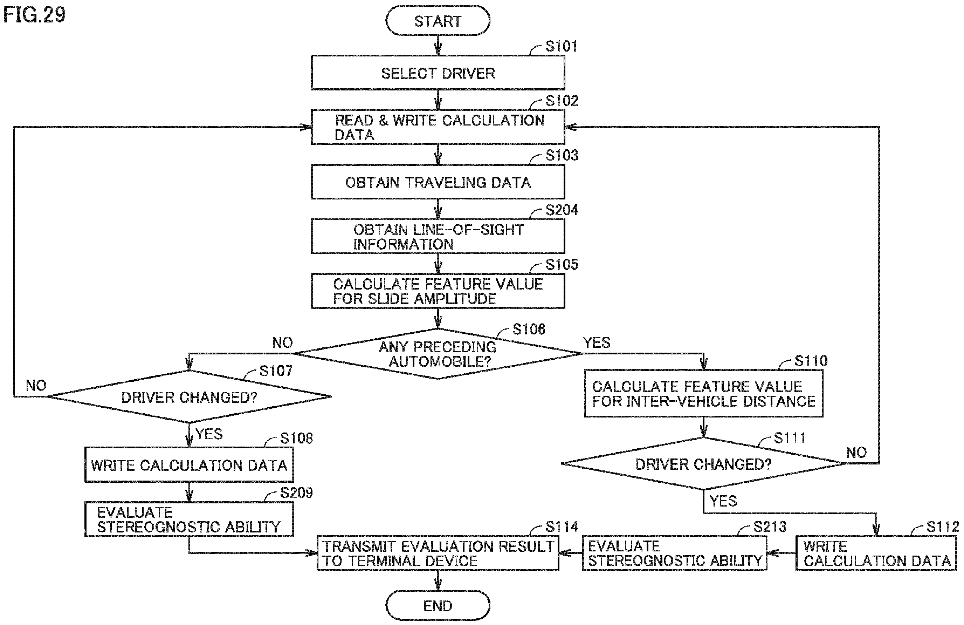

Fig. 29 is a flowchart of a process in a stereognostic ability evaluation method performed by processor 201 executing stereognostic ability evaluation program 203a of Fig. 24. The flowchart of the process shown in Fig. 29 corresponds to that shown in Fig. 18 with S204 added between S103 and S105, and S109 and S113 replaced with S209 and S213, respectively.

As shown in Fig. 29, after executing S101 to S103 in the same manner as in the first embodiment, processor 201 acquires line-of-sight information from line-of-sight/pupil sensor 212 in S204, and proceeds to S105. After executing S105 to S108 or S105, S106 and S110 to S112 in the same manner as in the first embodiment, processor 201 uses evaluation model 203c to evaluate subject Sb2's stereognostic ability in each of S209 and S213 based on a feature value for a slide amplitude and visual recognition information, and proceeds to S 114. As in the first embodiment, processor 201 performs S 114 and ends the process.

Thus, the stereognostic ability evaluation system, stereognostic ability evaluation device, stereognostic ability evaluation program, and stereognostic ability evaluation method according to the second embodiment can implement objective evaluation of stereognostic ability.

### [Third Embodiment]

In the first embodiment has been described a configuration in which a feature value is calculated from a position of a moving object as measured by a position measurement unit of a drive recorder. In a third embodiment will be described a configuration in which a feature value is directly measured by an acceleration sensor of a drive recorder.

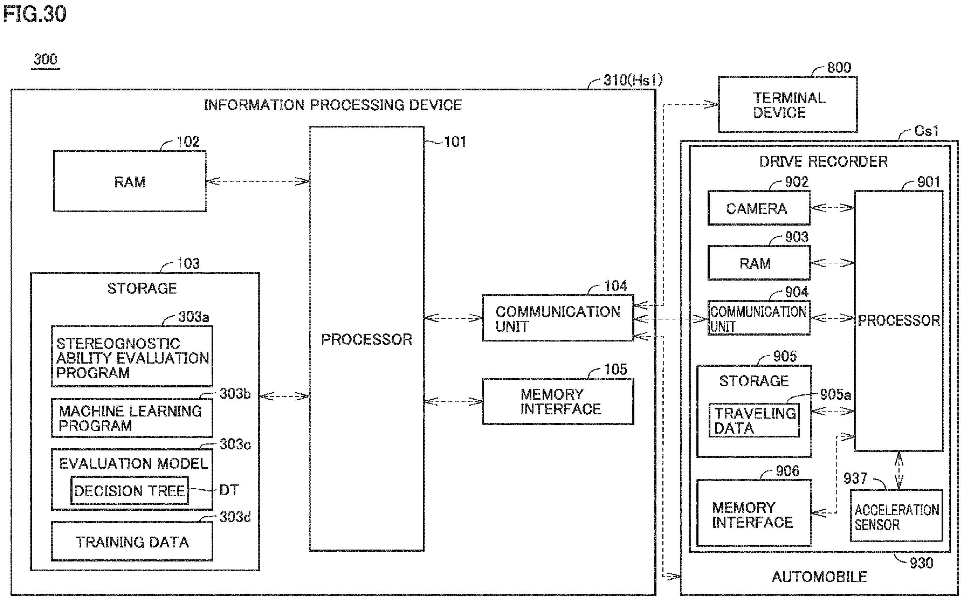

Fig. 30 is a diagram showing a hardware configuration of a stereognostic ability evaluation system 300 according to the third embodiment. An information processing device 310 shown in Fig. 30 has a configuration in which the Fig. 2 stereognostic ability evaluation program 103a, machine learning program 103b, evaluation model 103c, and training data 103d are replaced with a stereognostic ability evaluation program 303a, a machine learning program 303b, an evaluation model 303c, and training data 303d, respectively. A drive recorder 930 shown in Fig. 30 has a configuration in which position measurement unit 907 shown in Fig. 2 is replaced with an acceleration sensor 937. Acceleration sensor 937 measures, for example, an acceleration of automobile Cs1 in a direction (a lateral direction) orthogonal to a reference line extending in a direction in which automobile Cs1 travels. Acceleration sensor 937 may measure an acceleration of automobile Cs1 in a direction along the reference line. Except for this, stereognostic ability evaluation system 300 has a configuration similar to that of stereognostic ability evaluation system 100, and accordingly, will not be described repeatedly for that configuration.

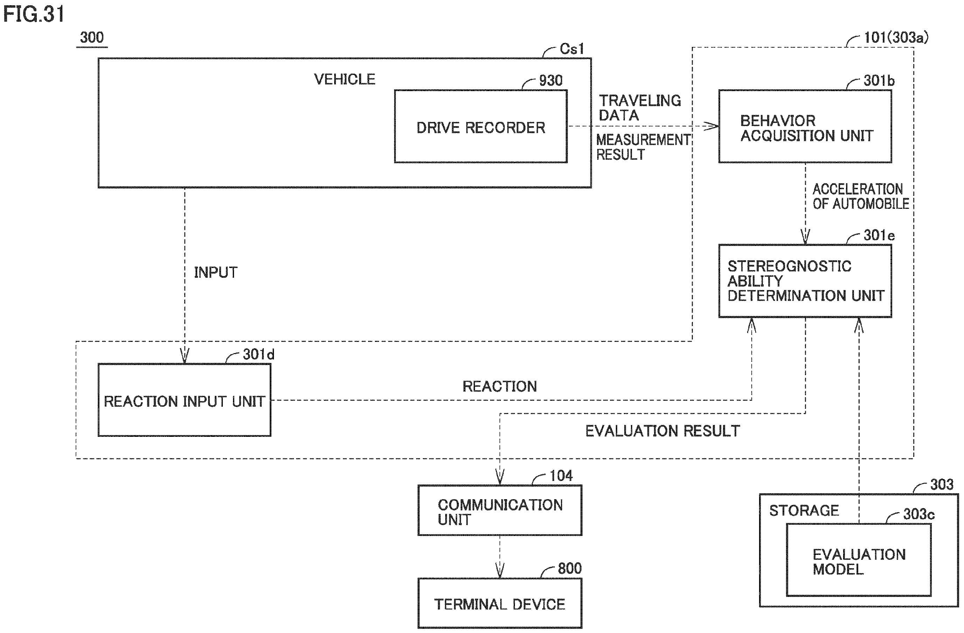

Fig. 31 is a functional block diagram of processor 101 of Fig. 30 showing a configuration for a function to evaluate stereognostic ability. In stereognostic ability evaluation system 300, processor 101 executes stereognostic ability evaluation program 303a stored in storage 103 to configure a module forming functional blocks referred to as a behavior acquisition unit 301b, a reaction input unit 301d, and a stereognostic ability determination unit 301e. Therefore, in place of processor 101 and stereognostic ability evaluation program 303a shown in Fig. 30, Fig. 31 shows functional blocks implemented thereby. These functional blocks will now be described below.

As shown in Fig. 31, behavior acquisition unit 301b acquires from drive recorder 930 a subject's traveling data, and a result of a measurement by acceleration sensor 937 as information about a behavior of automobile Cs1. Behavior acquisition unit 301b acquires an acceleration (for example, a slide acceleration) of automobile Cs1 from the traveling data and the measurement result. Behavior acquisition unit 301b outputs the acceleration of automobile Cs1 to stereognostic ability determination unit 301e. Behavior acquisition unit 301b may obtain information about a position of automobile Cs1 as the information about the behavior of automobile Cs1 from the traveling data of the subject and a positioning result of the position of automobile Cs1 received from drive recorder 930.

Reaction input unit 301d receives an input of an active reaction made by the subject to correspond to a three-dimensional position of automobile Cs1 as recognized by the subject. Reaction input unit 301d determines the reaction of the subject driving automobile Cs1, based on information input by the subject to the handle, accelerator pedal, brake pedal, etc. of automobile Cs1. Reaction input unit 301d outputs information about the reaction to stereognostic ability determination unit 301e.

Stereognostic ability determination unit 301e uses evaluation model 303c to determine the stereognostic ability of the subject from the acceleration of automobile Cs1. The information received from reaction input unit 301d about the reaction may be used to determine the stereognostic ability of the subject. As well as stereognostic ability determination unit 101e of the first embodiment, stereognostic ability determination unit 301e transmits an evaluation result of the stereognostic ability to terminal device 800 of the subject via communication unit 104.

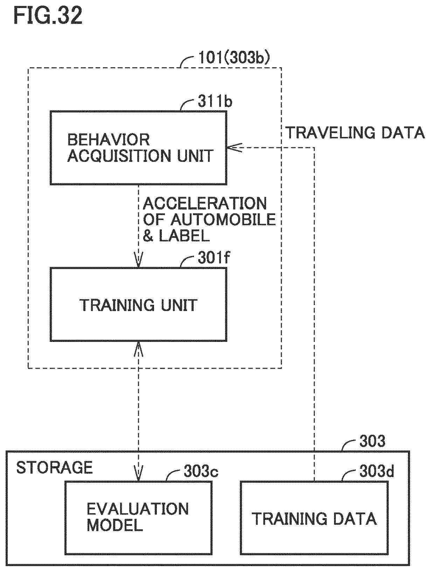

Fig. 32 is a functional block diagram of processor 101 of Fig. 30 showing a configuration of a machine learning function. In stereognostic ability evaluation system 300, processor 101 executes machine learning program 303b to configure a module forming functional blocks referred to as a behavior acquisition unit 311b and a training unit 301f. Accordingly, in place of processor 101 and machine learning program 303b shown in Fig. 30, Fig. 32 shows functional blocks implemented thereby. The functional blocks will now be described below.

As shown in Fig. 32, behavior acquisition unit 311b acquires traveling data from training data 303d. As well as behavior acquisition unit 301b of Fig. 31, behavior acquisition unit 311b acquires an acceleration of an automobile corresponding to the traveling data. Behavior acquisition unit 311b outputs the acceleration and a label attached to the traveling data to training unit 301f.

Training unit 301f uses the acceleration and the label received from behavior acquisition unit 311b to subject evaluation model 303c to machine learning to train evaluation model 303c.

Table 1 below indicates an example of correlation coefficients between each of feature values S0_s, S1_s and S2_s derived from a slide acceleration obtained by acceleration sensor 937 and each of feature values S0_s and AS1_s derived from a slide amplitude in a virtual space as obtained by the stereognostic ability evaluation system according to the second embodiment for a plurality of subjects. Note that each feature value indicated in Table 1 corresponds to a feature value indicated in Fig. 8 that is the same character string as the feature value.

**[Table 1]**

| | | acceleration sensor | | |
|---|---|---|---|---|
| | | S0_s | S1_s | S2_s |
| virtual space | S0_s | 0.73 | 0.35 | 0.14 |
| | AS1_s | 0.87 | 0.56 | 0.38 |

Regarding the feature values derived from the slide acceleration acquired by acceleration sensor 937, feature value S2_s is a segmental standard deviation of the slide acceleration acquired by acceleration sensor 937. Feature value S1_s is a segmental standard deviation of slide velocity, and is derived by integrating feature value S2_s over time once. Feature value S0_s is a segmental standard deviation of slide amplitude, and is derived by integrating feature value S1_s over time once (or integrating feature value S2_s over time twice). The slide acceleration acquired by acceleration sensor 937 can be obtained, for example, from a drive recorder of an automobile running a course of a driving school including an S-curve, a dogleg, a straight line, and the like.

Regarding the feature values derived from the slide amplitude in the virtual space, feature value S0_s is a segmental standard deviation of the slide amplitude. Feature value AS1_s is a segmental standard deviation of slide velocity in absolute value.

As indicated in Table 1, a positive correlation is observed between each of feature values S0_s, S1_s and S2_s and each of feature values S0_s and AS1_s derived from a slide amplitude acquired in a virtual space according to the second embodiment. Therefore, as well as feature values S0_s and AS1_s derived from the slide amplitude in the virtual space, feature values S0_s, S1_s and S2_s for the slide amplitude acquired by acceleration sensor 937 also allow objective evaluation of stereognostic ability.

When an interval between a reference object and a moving object is defined as a first distance and a distance (or a slide amplitude) between a reference line extending in a direction in which the moving object travels and the moving object is defined as a second distance, the present disclosure includes an embodiment of the following scope.

The presently disclosed stereognostic ability evaluation system evaluates a subject's stereognostic ability based on a behavioral change of a moving object moving as operated by the subject. The stereognostic ability evaluation system comprises a behavior acquisition unit and a stereognostic ability determination unit.

The stereognostic ability determination unit determines the stereognostic ability of the subject from at least one of a first feature value and a second feature value, the first feature value being relevant to a first distance between a reference object preceding the moving object in the direction in which the moving object travels and the moving object, the second feature value being relevant to a second distance between a reference line extending in the direction in which the moving object travels and the moving object. Further, the behavior acquisition unit acquires information about an acceleration of the moving object.

The first feature value and/or the second feature value are feature values derived from the information about the acceleration of the moving object. Further, the first feature value for the first distance may be derived from any one of a value of the acceleration in the direction of the first distance, a value obtained by integrating the acceleration in the direction of the first distance over time once, and a value obtained by integrating the acceleration in the direction of the first distance over time twice. Further, the second feature value for the second distance may be derived from any one of a value of the acceleration in the direction of the second distance, a value obtained by integrating the acceleration in the direction of the second distance over time once, and a value obtained by integrating the acceleration in the direction of the second distance over time twice.

While a configuration using an acceleration sensor has been described in the third embodiment, a gyro sensor (or an angular velocity sensor) may be used in place of the acceleration sensor, or both the acceleration sensor and the gyro sensor may be used together. When the gyro sensor is used, a feature value for determining a stereognostic ability may be derived from any one of a value of an angular velocity, a value obtained by integrating the angular velocity over time once, and a value obtained by integrating the angular velocity over time twice.

### [Exemplary Variation of Third Embodiment]

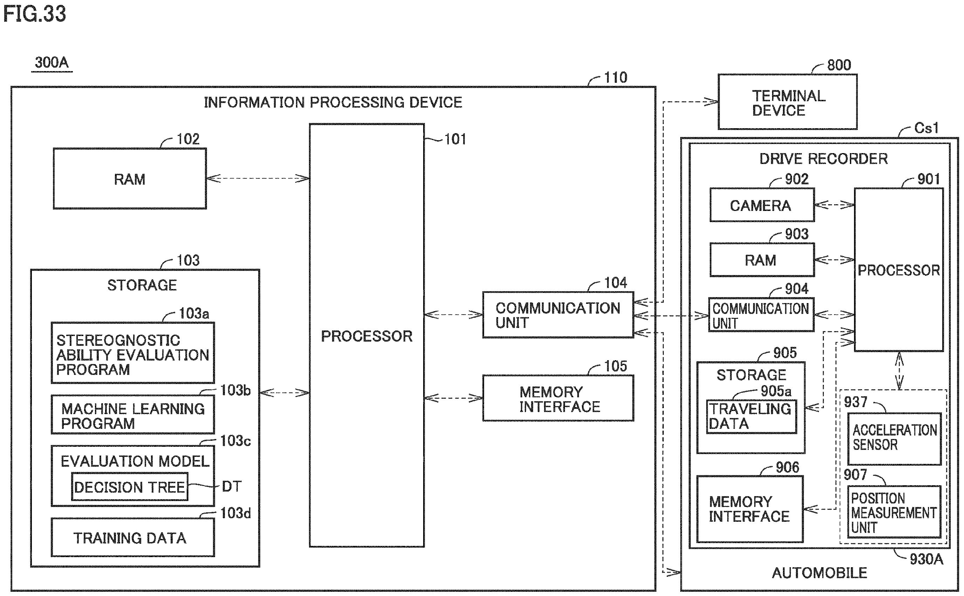

Fig. 33 is a diagram showing a hardware configuration of a stereognostic ability evaluation system 300A according to an exemplary variation of the third embodiment. Drive recorder 930 shown in Fig. 33 has a configuration in which acceleration sensor 937 shown in Fig. 30 is added to drive recorder 900 shown in Fig. 2. Except for this, stereognostic ability evaluation system 300A has a configuration similar to that of stereognostic ability evaluation system 100, and accordingly, will not be described repeatedly for that configuration.

Thus, the stereognostic ability evaluation system, stereognostic ability evaluation device, stereognostic ability evaluation program, and stereognostic ability evaluation method according to the third embodiment and the exemplary variation thereof can implement objective evaluation of stereognostic ability.

### [Fourth Embodiment]

While in the exemplary variation of the third embodiment has been described a configuration using data measured by an acceleration sensor or a position measurement unit of a drive recorder installed in an automobile, in the fourth embodiment will be described a configuration using data measured by a camera, an acceleration sensor, or a position measurement unit included in a portable mobile terminal such as a smartphone. An information processing device storing the a variety of types of programs may be included in the mobile terminal, or may be a device separate from the mobile terminal and communicating with the mobile terminal via a communication unit.

In the fourth embodiment, the mobile terminal includes a camera, an acceleration sensor, or a position measurement unit, and accordingly, a moving object is not limited to an automobile. The moving object may be any object insofar as it moves together with a subject and is positionally changed in response to an operation performed by the subject, and may for example be a bicycle, a motorcycle, an airplane, or a ship.

Thus, the stereognostic ability evaluation system, stereognostic ability evaluation device, stereognostic ability evaluation program, and stereognostic ability evaluation method according to the fourth embodiment can implement objective evaluation of stereognostic ability.

The objective evaluation of stereognostic ability described in implementations of the present disclosure, or the first to fourth embodiments, can be utilized in a variety of scenes. For example, the evaluation can be used as an indicator for development of higher cerebral functions in a human growth process. In particular, conventionally, a stereognostic ability has been evaluated based on solving a problem in a quiz format or on empirical rules in sports or the like. However, quantitatively evaluating a stereognostic ability using the stereognostic ability evaluation system, stereognostic ability evaluation device, stereognostic ability evaluation program, and stereognostic ability evaluation method described herein allows a more precise evaluation of stereognostic ability.

The stereognostic ability evaluation system, stereognostic ability evaluation device, stereognostic ability evaluation program, and stereognostic ability evaluation method described herein enable a continuous evaluation of a stereognostic ability during an activity of daily life such as driving an automobile. As a result, an objective evaluation of a stereognostic ability that cannot be achieved by a conventional evaluation method such as that in a quiz format performed within a determined period of time can be achieved.

Other examples of objective evaluation of stereognostic ability described herein include applying it to an assessment of cognitive disorders accompanying brain injury, mental diseases, dementia, and the like. In this example, using a virtual reality (or a virtual space) corresponding to a circumstance of a subject allows objective evaluation of stereognostic ability safely. In addition, it also becomes possible to confirm recovery of stereognostic ability through medication or a change such as an adverse effect or the like.

Still another example may include applying it to objectively defining an ability required for driving a moving object such as an automobile. Specifically, stereognostic ability can be defined as one such ability. In particular, an elderly person's impaired stereognostic ability due to aging may lead to an accident, and objective evaluation of stereognostic ability is also important in determining aptitude for driving. In addition, by objectively evaluating the stereognostic ability of a subject other than elderly people, such as an unskilled driver, a significantly fatigued driver and an inattentive driver, whether the subject has a low stereognostic ability can be early detected. As a result, it is possible to prevent an accident by alerting the subject that the subject has a low stereognostic ability. Note that a subject whose stereognostic ability can be objectively evaluated is not limited to a driver of an automobile, and includes an operator of a moving object that can travel in a straight line or a curved line. Examples of the moving object that can travel in a straight line and a curved line include bicycles, motorcycles, airplanes, and ships. Furthermore, objective evaluation of stereognostic ability can also be an indicator for a design of a moving object equipped with an auxiliary function to compensate for an impaired stereognostic ability.

The embodiments disclosed herein are also contemplated to be combined within a scope without contradiction, as appropriate, and implemented. It should be understood that the embodiments disclosed herein are illustrative and not restrictive in any respect. The scope of the present disclosure is defined by the terms of the claims, rather than the description above, and is intended to encompass any modifications within the meaning and scope equivalent to the terms of the claims.

### REFERENCE SIGNS LIST

100, 100A, 200, 300, 300A stereognostic ability evaluation system, 101, 201, 601, 901 processor, 101b, 111b, 201b position acquisition unit, 101d, 201d, 301d reaction input unit, 101e, 201e, 301e stereognostic ability determination unit, 101f, 301f training unit, 102, 202, 602, 903 RAM, 103, 203, 603, 905 storage, 103a, 203a, 303a stereognostic ability evaluation program, 103b, 203b, 303b machine learning program, 103c, 203c, 303c evaluation model, 103d, 203d, 303d training data, 104, 204, 604, 904 communication unit, 105, 205, 605, 906 memory interface, 110, 110A, 310 information processing device, 201a moving-object display unit, 201c visual recognition determination unit, 210 stereognostic ability evaluation device, 211 electronic display, 212 pupil sensor, 301b, 311b behavior acquisition unit, 600 training device, 800, 820 terminal device, 900 drive recorder, 902 camera, 905a traveling data, Ap accelerator pedal, Bp brake pedal, Ca1, Ca2 preceding automobile, Cd control device, Cs1, Cs2 automobile, DT, DT1-DT6 decision tree, Hd handle, Hs1, Hs2, Hs11 housing, NW Network, Rb attachment band, Sb1, Sb2 subject.

## Claims

1. A stereognostic ability evaluation system that evaluates a stereognostic ability of a subject based on a positional change of a moving object moving as operated by the subject, comprising:
a position acquisition unit that acquires information about a position of the moving object; and
a stereognostic ability determination unit that determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

2. The stereognostic ability evaluation system according to claim 1, wherein the second feature value includes a feature value derived from a value obtained by differentiating the second distance with time at least once.

3. The stereognostic ability evaluation system according to claim 1, wherein the second feature value includes a feature value derived from a value obtained by differentiating the second distance with time at least twice.

4. The stereognostic ability evaluation system according to any one of claims 1 to 3, wherein the first feature value includes a feature value derived from a value obtained by differentiating the first distance with time at least twice.

5. The stereognostic ability evaluation system according to any one of claims 1 to 4, wherein the stereognostic ability determination unit determines the stereognostic ability from at least one of the first feature value and the second feature value using a trained specific model.

6. The stereognostic ability evaluation system according to claim 5, wherein
the specific model includes a decision tree with the stereognostic ability as an objective variable, and
the decision tree has an explanatory variable including at least one of the first feature value and the second feature value.

7. The stereognostic ability evaluation system according to claim 6, wherein the explanatory variable includes information about an attribute of the subject.

8. The stereognostic ability evaluation system according to any one of claims 1 to 7, further comprising a movement recording device mounted on the moving object, wherein
the movement recording device includes
a camera that generates video data as viewed from the moving object in the direction in which the moving object travels, and
a position measurement unit that measures the position of the moving object, and
the position acquisition unit acquires information about the position of the moving object from the video data and a measurement result by the position measurement unit.

9. The stereognostic ability evaluation system according to any one of claims 1 to 7, the moving object being provided by a virtual reality, the stereognostic ability evaluation system further comprising:
a virtual reality headset including an electronic display for displaying a video of the virtual reality; and
a moving-object display unit that causes the electronic display to display the video of the moving object while the moving object moves in the virtual reality, wherein the position acquisition unit acquires a position of the moving object in the virtual reality as displayed by the moving-object display unit.

10. A stereognostic ability evaluation system that evaluates a stereognostic ability of a subject based on a behavioral change of a moving object moving as operated by the subject, comprising:
a behavior acquisition unit that acquires information about a behavior of the moving object; and
a stereognostic ability determination unit that determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.

11. The stereognostic ability evaluation system according to claim 10, wherein
the behavior acquisition unit acquires information about a position of the moving object, and
at least one of the first feature value and the second feature value is derived from information about the position of the moving object.

12. The stereognostic ability evaluation system according to claim 10, wherein
the behavior acquisition unit acquires information about an acceleration of the moving object, and
the stereognostic ability determination unit determines the stereognostic ability from the second feature value.

13. The stereognostic ability evaluation system according to claim 12, wherein the second feature value is derived from the acceleration of the moving object.

14. The stereognostic ability evaluation system according to claim 12 or 13, further comprising a movement recording device mounted on the moving object, wherein
the movement recording device includes
a camera that generates video data as viewed from the moving object in the direction in which the moving object travels, and
an acceleration sensor that measures the acceleration of the moving object, and
the behavior acquisition unit acquires information about the acceleration of the moving object from the video data and a measurement result by the acceleration sensor.

15. The stereognostic ability evaluation system according to any one of claims 1 to 14, further comprising:
a terminal device of the subject; and
a communication unit that transmits a determination result from the stereognostic ability determination unit to the terminal device.

16. A stereognostic ability evaluation device that evaluates a stereognostic ability of a subject based on a positional change of a moving object moving as operated by the subject, comprising:
a position acquisition unit that acquires information about a position of the moving object;
a stereognostic ability determination unit that determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels; and
a housing that accommodates the position acquisition unit and the stereognostic ability determination unit.

17. A stereognostic ability evaluation device that evaluates a stereognostic ability of a subject based on a behavioral change of a moving object moving as operated by the subject, comprising:
a behavior acquisition unit that acquires information about a behavior of the moving object;
a stereognostic ability determination unit that determines the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels; and
a housing that accommodates the behavior acquisition unit and the stereognostic ability determination unit.

18. A stereognostic ability evaluation program executed by a computer to cause the computer to configure a stereognostic ability evaluation device to evaluate a stereognostic ability of a subject based on a positional change of a moving object moving as operated by the subject, the stereognostic ability evaluation device including:
a position acquisition unit that acquires information about a position of the moving object; and
a stereognostic ability determination unit that evaluates the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

19. A stereognostic ability evaluation program executed by a computer to cause the computer to configure a stereognostic ability evaluation device to evaluate a stereognostic ability of a subject based on a behavioral change of a moving object moving as operated by the subject, the stereognostic ability evaluation device including:
a behavior acquisition unit that acquires information about a behavior of the moving object; and
a stereognostic ability determination unit that evaluates the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.

20. A stereognostic ability evaluation method for evaluating a stereognostic ability of a subject based on a positional change of a moving object moving as operated by the subject, comprising:
acquiring information about a position of the moving object; and
determining the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being derived from a second distance between a reference line and the moving object, the reference line extending in the direction in which the moving object travels.

21. A stereognostic ability evaluation method for evaluating a stereognostic ability of a subject based on a behavioral change of a moving object moving as operated by the subject, comprising:
acquiring information about a behavior of the moving object; and
determining the stereognostic ability from at least one of a first feature value and a second feature value, the first feature value being derived from a value obtained by differentiating a first distance between a reference object and the moving object with time at least once, the reference object preceding the moving object in a direction in which the moving object travels, the second feature value being relevant to a second distance between a reference line and the moving object, the reference line extending in the direction in which the moving object travels.
